# EUROPEAN PATENT APPLICATION

(11) **EP 3 533 455 A1**
(43) Date of publication of application: **04.09.2019**
(21) Application number: 18000198.4
(22) Date of filing: 01.03.2018
(51) Int. Cl.: A61K 38/06, A61K 38/07, A61K 38/05, A61K 31/69, A61P 43/00, A61K 31/00

(54) **INHIBITION OF PROTEOLYTIC ACTIVITY IN THE TREATMENT OF MITOCHONDRIOPATHIES**

(71) Applicant: International Institute of Molecular and Cell Biology in Warsaw, 02-109 Warsaw (PL); University of Warsaw, 00-927 Warszawa (PL)
(72) Inventor: The designation of the inventor has not yet been filed
(74) Representative: Banse & Steglich Patentanwälte PartmbB

(57) **Abstract**

The present invention relates to the treatment of mitochondriopathies. Specifically, the invention relates to inhibitors of proteolytic activity for use in a method of treating or preventing a mitochondriopathy caused by at least one genetic mutation, especially at least one mutation in a nuclear gene. Furthermore, the invention relates to a method for treating or preventing a mitochondriopathy comprising the reduction of cellular proteolytic activity. In addition the present invention relates to respective uses and composition.

## Description

### Field of invention

The present invention relates to the treatment of mitochondriopathies. Specifically, the invention relates to inhibitors of proteolytic activity for use in a method of treating or preventing a mitochondriopathy caused by at least one genetic mutation, especially at least one mutation in a nuclear gene. Furthermore, the invention relates to a method for treating or preventing a mitochondriopathy comprising the reduction of cellular proteolytic activity. In addition the present invention relates to respective uses and composition.

### Background

Eukaryotic cells are compartimented into numerous organelles, surrounded by membranes which perform specific functions within the cells. Mitochondria are organelles which are crucial for numerous cellular processes, most importantly including energy metabolism, and programmed cell death (apoptosis). Furthermore, mitochondria are involved in the metabolic pathways of lipids and iron and other classes of molecules.

Mitochondria are constituted by an outer mitochondrial membrane, an inner membrane, which is folded in cristae to increase its surface area, the intermembrane space between the aforementioned membranes and the mitochondrial matrix, surrounded by the inner membrane. Human mitochondria are comprise about 1500 different proteins, which can be categorised into membrane proteins residing in the inner or outer membrane, or soluble proteins localised in the intermembrane space or the matrix. About 1% of the mitochondrial proteins are encoded by mitochondrial DNA and synthesised by ribosomes in the mitochondrial matrix. The respective proteins are primarily the highly hydrophobic transmembrane proteins of the respiratory chain residing in the inner membrane. However, the vast majority of mitochondrial proteins is nuclear encoded and synthesized as pre-proteins by cytosolic ribosomes.

Nuclear encoded proteins localized in the mitochondria comprise targeting signals that facilitate their intramitochondrial sorting. A first class of pre-proteins consists of pre-proteins with N-terminal sorting sequences which are typically cleaved after import. Typically, pre-proteins from this group are sorted to the matrix, the intermembrane space or the inner membrane. A further group of pre-proteins, consisting of proteins of integral proteins of the outer and inner membrane, comprises internal sorting sequences. Lastly, proteins sorted to the intermembrane space typically comprise internal cysteine comprising targeting signals.

The respective mechanisms were investigated in yeast in great detail, thus it is referred to the respective yeast protein nomenclature. However, homologs and orthologs of the yeast protein exist in mammals including humans. Thus, herein the reference to a protein in yeast nomenclature also refers to the respective homologs in mammals, especially in humans.

The aforementioned pre-proteins are directed from their cytosolic site of synthesis to their final destination via four basic pathways. Virtually all mitochondrial proteins are imported by the general protein import complex in the membrane, the TOM complex. The integral membrane protein Tom40 constitutes a channel for free protein translocation across the outer membrane and thus forms the central component of the complex. Furthermore, several receptor or proteins direct proteins from the cytosol to the Tom40. While Tom20 functions for proteins with pre-sequences, Tom70 show receptor or site for pre-proteins with multiple internal targeting signals. Both initial receptors transfer the pre-proteins to the central pore via the central receptor Tom22. Although, Tom40 represents the central important pore, at least 2 different mechanisms for translocation through this pore exist. Pre-proteins carrying an N-terminal pre-sequence are translocated as loosely folded polypeptide chains through the Tom40 pore and bind to the intermembrane space Tim (translocase of the inner mitochondrial membrane), while multiple internal targeting signals are translocated as a loop, wherein the ends of the pre-proteins is still in the cytosol, while the middle portion has already reached the intermembrane space.

Pre-sequence carrying proteins are further translocated by the Tim23 complex, consisting of the components Tim50, which fulfils the receptor function in the intermembrane space for the translocase complex, the channel-forming Tim23 protein, and Tim17 which recruits the matrix exposed import motor PAM. The interaction of the inner membrane translocase complex with the Tom complex of the outer membrane is facilitated by Tim21.

Upon insertion into the Tim23 pore, the import pathways deviate into the complete translocation through the pore of matrix proteins and a lateral release of inner membrane proteins harbouring a single transmembrane domain into the inner membrane. Import into the matrix is driven by the PAM complex comprising Tim44 which binds the chaperone Hsp70 that provides import driving energy by ATP hydrolysis.

Inner membrane proteins with multiple membrane spanning domains, which thus have more hydrophobic properties, take a different import route. To prevent aggregation these proteins are directed to the Tom complex in a chaperone bound state, transferred to the Tom channel via Tom70 oligomers and again bound to a chaperone complex, the Tim9-Tim10 complex, at the intermembrane space side of the Tom channel to finalize translocation through the outer membrane. Alternatively, the Tim8-Tim13 complex acts as a chaperone complex in the intermembrane space. Notably, mutations in the human Tim8 proteins have been identified as the cause of Mohr-Tranebjaerg / deafness dystonia syndrome.

At the inner membrane side of the intermembrane space, Tim54 again acts as a receptor and transfers the pre-protein to Tim22, which constitutes the channel forming entity of the carrier translocase complex of the inner membrane (also termed Tim22 complex). In the final step of this pathway, the carrier proteins inserted in Tim22 pore are laterally released into the inner membrane.

Intermembrane space proteins, like the afore mentioned Tim8, 9, 10, 13 are often proteins that comprise cysteine rich motifs. Most of these proteins are bound to MIA40 upon emergence of the translocating proteins at the intermembrane space side of the TOM complex. In cooperation with Erv1 and Hot13, Mia40 facilitates the folding of the intermembrane space proteins in a process that incudes reduction of the cysteine moieties under formation of disulfide bonds. By means of this oxidative folding facilitated by MIA40 the much you are protein is trapped in the into membrane space.

Similar to the integral membrane proteins of the inner mitochondrial membrane, also the membrane proteins of the outer mitochondrial membrane are imported via 2 distinct pathways. Beta barrel proteins, like porin or Tom40 itself, are translocated the through the Tom40 pore into the intermembrane space, where the Tim9-Tom10 or Tim8-Tim13 chaperone complexes bind the precursor protein which guide them to the sorting and assembly machinery for mitochondria outer membrane proteins (SAM complex) comprising the core components SAM50 and SAM35. After binding of the pre-proteins by SAM35 in a receptor or like action, the proteins are inserted into the SAM50 channel and laterally released into the membrane. For alpha helical proteins of the outer membrane, several imports pathways that may involve or may not involve the Tom complex has been suggested.

A further assembly factor involved in mitochondrial diseases is RESA1 (Respiratory Chain Assembly1, also termed COA7 or C1orf163). A knockdown of RESA1 leads to reduced levels of respiratory chain complexes I, III and IV (Kozjak-Palovic et al., 2013). Thus, it has been concluded that RESA1 is an assembly factor for respiratory chain complexes. The protein is located in the intermembrane space. Since the protein comprises several cysteine rich regions, it seems likely that the protein itself is a substrate of the MIA complex.

Bragoszewski et al. 2013, further identified a role of cytosolic degradation in the biogenesis of mitochondria proteins. Unexpectedly, ubiquitinated species of precursor proteins targeted to the inter membrane space were identified in cells even under conditions where the proteasome was fully active. While it was previously known that cytochrome C and EndoG pre-proteins may be degraded by the proteasome, the study revealed that the whole class of MIA translocation dependent pre-proteins is controlled by proteasomal degradation.

Furthermore, conformationally destabilized proteins are released from mitochondria in a size-limited manner through the Tom40 as an escape gate. It has therefore been proposed that the mitochondrial proteome is not only regulated by the import and degradation of proteins but also by their retro-translocation to the external cytosolic location were the proteins are cleared by proteasomes (Bragoszewski et al. 2015).

Generally, aggregated or damaged cell proteins are degraded by the proteasomes, which thus acts as a control system. Furthermore, degradation is used by the cell to regulate the half-life of proteins that play major roles in regulatory process in various aspects of cell function. For the mitochondrial protein APOPT1 it could be shown by inhibition experiments using a small molecule proteasome inhibitor that under standard culturing conditions, while the *APOPT1* gene was transcribed, the protein was degraded by the proteasome. However, under oxidative stress conditions APOPT1 protein levels increased due to inhibition of the proteasomal degradation. Degradation of proteins by the proteasome is triggered by attachment of ubiquitin to the respective proteins. Ubiquitin is a 76-amino acid protein. Ubiquitination is facilitated by an enzymatic reaction requiring Ub-activating (E1), Ub-conjugating (E2) and Ub-ligating (E3) enzymes acting sequentially. In total, more than four ubiquitin units must be linked to the target protein for proteasome degradation (Grigoreva et al. 2016).

Proteasomes are abundant structures in the cell and are located in the cytoplasm and nuclei. The term proteasome usually refers to the 26S proteasome and includes a 20S core catalytic complex with 19S regulatory subunits. The S19 regulatory particle guides ubiquitinated proteins into the 20S particle. The 20S core complex houses the proteolytic chamber. The 20S complex includes four ring structures and three proteolytic activities in the beta rings specifically caspase-like (C-L), trypsin-like (T-L) and chymotrypsin-like (CT-L) catalytic proteases (Grigoreva et al. 2016).

Similar to kinases, the components of the ubiquitin system are often dysregulated, leading to a variety of diseases, including cancer, neuro-degeneration, or auto-immune disorders, making them attractive drug targets. Thus, a multitude of inhibitors of the ubiquitin system, especially inhibitors of the proteasome, were designed and evaluated in the recent years. A first proteasome inhibitor from the boronate compound family, bortezomib (Velcade, Millennium Pharmaceuticals), a proteasome from the epoxyketone family, such as carfilzomib, a proteasome inhibitor from the peptide boronic acid family, such as ixazomib (Millenium Pharmaceuticals). As representative from the structural family of beta lactones, the drug marizomib is currently investigated in a phase III trial for the treatment of glioblastoma. A proteasome inhibitor which is commonly used for *in vitro* studies is the synthetic peptide MG132.

In addition to inhibitors of the proteasome, inhibitors of the ubiquitin activating enzymes (UBEs or E1 enzymes), the E2 ubiquitin conjugating enzymes, and ubiquitin E3 ligase family members were investigated in animal models or clinical trials (Huang & Dixit, 2016).

Given their crucial function for numerous cellular processes, a malfunction of mitochondria due to mutations residing in mitochondrial proteins results in numerous fatal diseases. Mitochondrial diseases are one of the most common types of inherited metabolic disorder, which show unprecedented variability in clinical presentation and can manifest at any age in any organ. Despite dramatic improvements in the genetic and metabolic diagnosis of these severe progressive diseases, there are still no curative treatments (Suomalained & Battersby, 2017). Predominantly, mitochondrial diseases are caused by defects in respiratory chain. Naturally, this diseases may be divided into conditions caused by mutation in the nuclear genome or mutations in the mitochondrial genome.

The first underlying nuclear gene mutation of a mitochondrial disorder was identified in SDHA in a patient with complex II deficiency and Leigh syndrome. Based on recent compilations (Calvo & Mootha, 2010; Koopman et al. 2012) and disease databases (http://omim.org), there are now over 110 nuclear genes that are known to be mutated in mitochondriopathies such as respiratory chain diseases. This number is rapidly expanding. The genes that have been identified so far can be organized into the five groups: oxidative phosphorylation subunits, mtDNA maintenance and expression, oxidative phosphorylation biogenesis an regulation, nucleotide transport and synthesis and membrane dynamics and composition.

Respective genes of oxidative phosphorylation subunits are for example the Complex I genes NDUFA1, NDUFA2, NDUFA9, NDUFA10, NDUFA11, NDUFA12, NDUFB3, NDUFB9, NDUFS1, NDUFS2, NDUFS3, NDUFB9, NDUFS1, NDUFS2, NDUFS3, NDUFV2; the complex II genes SDHA, SDHB, SDHC, SDHD; the complex III genes UQCRB, UQCRQ; the complex IV genes C0X412 and COX6B1; and the complex V gene ATP5E. Respective genes of relating to mtDNA maintenance and expression are for example TWINKLE, MTFMT, GFM1, LRPPRC,MPV17, MRPS16, MRPS22, POLG, POLG2, TRMU, TSFM, TUFM,C12orf65, MTPAP, MRPL3, SARS2,YARS2, HARS2, MARS2, AARS2,RARS2, EARS2, DARS2, TAC01,MT01, RMND1, PNPT1, PUS1. Respective genes relating to oxidative phosphorylation biogenesis an regulation are for example the complex I genes NDUFAF1, NDUFAF2, NDUFAF3, NDUFAF4, NDUFAF5, NDUFAF6, ACAD9, FOXRED1, NUBPL; the complex II genes SDHAF1, SDHAF2 Complex III: BCS1L, HCCS, TTC19, the complex III genes COX10, COX15, ETHE1, FASTKD2, SCO1, SCO2, SURF1, COX14, COA5, the complex V genes ATPAF2, TM EM 70; the Fe-S genes ABCB7, FXN, ISCU, NFU1, BOLA3, GLRX5 and other genes including DNAJC19, GFER, HSPD1, SPG7, TIMM8A, AIFM1, and AFG3L2. Respective genes relating to nucleotide transport and synthesis are for example DGUOK, RRM2B, SLC25A3, ANT1, SUCLA, SUCLG1, TK2, TYMP. Respective genes relating to membrane dynamics and composition are for example ADCK3, AGK, COQ2, COQ6, COQ9, DRP1, MFN2, OPA1, PDSS1, PDSS2, TAZ and SERAC1 (Vafail & Mootha, 2012).

Notably, as mentioned above, the human TIMM8A or DDP gene is an intermembrane space subunit of the mitochondrial protein translocase (Roesch et al, 2002; Koehler et al., 1999) and mutations within this protein have been identified as the cause of Mohr-Tranebjaerg and Jensen Syndrome which are associated with deafness-dystonia-optic atrophy syndrome (Tranebjaerg et al, 2000).

One of the most common mitochondrial respiratory-chain defects in humans is cytochrome c oxidase (COX, complex IV) deficiency. Like Mohr-Tranebjaerg, also this defect may be caused by mutations in a mitochondrial biogenesis factor. Mutations in the COA6 gene have been identified in patients with cytochrome c oxidase deficiency, suffering from conditions like severe infantile encephalomyopathy, hypertrophic obstructive cardiomyopathy. Mechanistic studies reveal that the nuclear encoded COA6 gene product functions as an assembly factor for complexIV, which consists of nuclear and mitochondrial encoded subunits. Interestingly pathogenic mutation in COA6 does not affect its import into mitochondria but impairs its maturation and stability (Stroud et al., 2015).

Regarding the above mentioned RESA1 gene, mutations have been identified in patients suffering from mitochondrial leukoencephalopathy (Martinez Lyons et al. 2016). Generally, leukoencephalopathies are diseases affecting the white matter of the central nervous system. In the case investigated by Martinez Lyons and colleagues, the patient showed clinical signs of psychomotor delay, demyelinating sensorimotor neuropathy, dysarthria, dysmetria, ataxic gait and hyporeflexia, mild cognitive impairment. Brain and spinal cord MRI showed mild extension of signal abnormalities and extensive cavitations in the cerebral white matter; the cerebellum and brainstem.

Over the past years a variety of approaches for the treatment of mitochondrial diseases has been investigated in animal studies or clinical studies in humans. Many intervention strategies were based on boosting mitochondrial biogenesis and activating nutrient sensors using genetic and small-molecule approaches. For example, peroxisome proliferator-activated receptor (PPAR) agonist bezafibrate, a ketogenic diet and 5-aminoimidazole-4-carboxamide ribonucleotide (AICAR; an agonist of AMP-activated kinase) all promoted mitochondrial biogenesis, induced lipid oxidation, and improved muscle metabolism in mitochondrial myopathy models. In addition, application of vitamin B3 (nicotinamide riboside) or inhibitors of the NAD'-consuming enzyme PARP increased the ratio between oxidized NAD' and its reduced form (NADH), which reflects the redox state of the cell, induced sirtuins and PPARy co-activator 1 a (PGC1a) and promoted mitochondrial biogenesis in mitochondrial disease, and decreased the disease-related myopathy symptoms. Furthermore it could be shown that rapamycin, an inhibitor of mTORC 1, reduced brain manifestations in complex I-deficient mice (Suomalain & Battersby, 2017). In addition, dichloroacetate (DCA), an activator of pyruvate dehydrogenate complex or coenzyme Q10 and its analog idebenone have been evaluated in human clinical trials for the treatment of congenital mitochondrial disorders (Kerr, 2010). However, it still remains to be clarified whether all tissues benefit in the long-term from these treatments, which aim to promote biogenesis and/or metabolic activity of the primary affected organelle.

Further treatments for mitochondrial diseases are suggested in prior patent applications:
WO 2006/021413 A1 suggests the use of alpha-keto carbonyl calpain inhibitors as inhibitors of cell damage by oxidative stress through free radicals. Cell damage by free radicals has been recognized to be involved in Kearns-Sayre syndrome, mitochondrial encephalomyopathy-lactic-acídosís-stroke like episodes (MELAS), myoclonic epilepsy and ragged-red-fibers (MERRF), Leber hereditary optic neuropathy (LHON), Leigh's syndrome, neuropathy-ataxia-retinitis pigmentosa (NARP) and progressive external opthalmoplegia (PEO) and as such they are useful for the preparation of a medicament for the treatment of mitochondrial disorders and neurodegenerative diseases, where elevated levies of oxidative stress are involved. The disclosed deficiencies are all caused by mutations in the mitochondrial genome (Wong, 2012). However, while an effect on damages caused by free oxigen radicals is also suggested, no general effect on mitochondrial diseases caused by mutations in nuclear genes is disclosed.

WO 01/02123589 A2 suggests the use of ubiquitine protease modulators for the treatment of mitochondrial encephalomyopathy, including Leigh syndrome. Mitochondrial encephalomyopathies may be caused be mutations in the mitochondrial genes (Wong, 2012). The document does not disclose a treatment for mitochondrial diseases caused by mutations in nuclear genes.

In summary, diseases caused by mutations in nuclear genes encoding mitochondrial proteins presently lack an adequate treatment. Since, the respective diseases are rare diseases, there seems little financial incentive to develop new treatments for each single disease.

### Problem underlying the invention

In view of the prior art, it was the general problem underlying the present invention to provide active agents, compositions, methods and uses to overcome the above-mentioned disadvantages of the prior art. Especially, agents, compositions and methods suitable for treating or preventing mitochondriopathies caused by a genetic mutation, especially mitochondriopathies caused by a mutation in a nuclear gene, should be provided. In a more specific aspect, active agents, compositions, methods and uses for the treatment of leukoencephalopathies should be provided. Furthermore, the agents and compositions should easily accessible, conveniently administrable, safe and easy to manufacture.

### Disclosure of the invention

Surprisingly, it was found that the problem underlying the invention is solved by the inhibitor of proteolytic activity, compositions, uses and methods according to the claims. Further embodiments of the invention are outlined throughout the description.

The inventors have found, that mutations in nuclear encoded mitochondrial proteins, such as for example RESA1, result in reduced levels of said proteins in mitochondria. Specifically it could be shown that cells transfected with plasmids encoding a RESA1 with the amino acid exchange tyrosine 137 cysteine or encoding a RESA1 with a deletion in exon 2 showed reduced amount of RESA1 in the mitochondria of the transfected cells. The mutant proteins exhibited a faster degradation in the cell. Notably, these mutations correspond to the nuclear mutations of a patient mitochondrial leukoencephalopathy and cytochrome c oxidase deficiency (Martinez Lyons et al. 2016). In accordance with these observation, the RESA1 protein was absent in fibroblasts of the patient examined by Martinez Lyons and colleagues.

As evidenced by the examples of the present application, the inventors surprisingly found that already the substitution of one amino acid in the mutated RESA1 mutants exhibited a pronounced import defect of the protein into mitochondria. The defect was even more pronounced for the mutant with a deletion in exon 2. Thus, the increased degradation in the cells observed for mutant RESA1 can be attributed to an increased cytosolic degradation of the mutant proteins due to a slower import into the mitochondria. It was further observed, that the degradation of newly synthesized mutated RESA1 can be rescued by proteasome inhibition.

It was further shown by the inventors, that subjecting patient cell lines harbouring the RESA1 mutants comprising a tyrosine 137 cysteine and a deletion in exon 2 with the proteasome inhibitors MG132, bortenzomib or carfilzomib did not only rescue the RESA1 levels in the mitochondria of these cells. Surprisingly, also an increase in the levels of proteins like COX6B and COX6A, components of Complex IV was observed upon administration of the protease inhibitors. Notably, reduced levels of Complex IV components are known causative for mitochondriopathies.

Thus, the invention generally relates to uses and methods wherein proteolytic activity is decreased for the treatment or prevention of a mitochondriopathy caused by at least one genetic mutation.

Thus, in first aspect, the invention relates to an inhibitor of proteolytic activity for use in a method of treating or preventing a mitochondriopathy caused by at least one genetic mutation.

Within the context of the present invention, an "inhibitor of proteolytic activity" may be any compound that reduces directly or indirectly the activity of a protease within the cell. Thus, the inhibitor of proteolytic activity may be any compound that reduces or abolishes the transcription or translation of a gene encoding a protease that forms a subunit or otherwise contributes to or regulates the activity of proteolytic activity within a cell. Thus, the inhibitor of proteolytic activity may also be any compound that inhibits regulatory elements that themselves increase proteolytic activity, or any compound that increases the activity of regulatory elements that themselves inhibit the activity of proteolytic activity in the cell. The "inhibitor of proteolytic activity" within the meaning of the present invention may not abolish the proteolytic activity totally but may decrease the proteolytic activity.

The inhibitor of proteolytic activity may inhibit proteolytic activity anywhere in cell, thus in any organelle or in the cytosol.

In a highly preferred embodiment, the inhibitor of proteolytic activity inhibits the proteolytic activity of proteasomes. The term "proteolytic activity" herein preferably refers to the degradation of proteins.

Within the context of the present invention, "treating or preventing" a mitochondriopathy relates to the application of an inhibitor of proteolytic activity as described herein, for (a) preventing the mitochondriopathy or symptom thereof from occurring in a subject which may be predisposed a mitochondriopathy due to a genetic mutation, disease or symptom thereof, but has not yet been diagnosed as having it; (b) inhibiting the mitochondriopathy symptoms, i.e. arresting its development; or (c) relieving or eliminating the mitochondriopathy or disease symptoms, i.e. causing regression of the mitochondriopathy, disease or symptoms thereof.

In a preferred embodiment, the mitochondriopathy is caused by at least one mutation in at least one nuclear gene.

Within the context of the present disclosure the term "nuclear gene" refers to genes encoded by the DNA comprised in the cell nucleus. The term "nuclear mutation" refers to a mutation in a nuclear gene. Likewise, the term "mitochondrial gene" refers to genes encoded by the DNA comprised in the mitochondria of the cell. The term "mitochondrial mutation" refers to a mutation in a mitochondrial gene.

A "genetic mutation" according to the present invention relates to an exchange or deletion of a nucleotide of a gene. Furthermore, the mutation may be an introduction of an additional nucleotide into the gene. The mutation may be in an intron or an exon. In a highly preferred embodiment, the genetic mutation according to the present invention introduces a deletion or substitution of an amino acid transcribed from the mutated gene in relation to the wild type gene.

In a preferred embodiment, the nuclear gene encodes a mitochondrial protein selected from a mitochondrial outer membrane protein, a mitochondrial intermembrane space protein, a mitochondrial inner membrane protein, a respiratory chain complex protein, and/or a mitochondrial matrix protein. More preferably, the gene encodes a protein of the mitochondrial inner membrane protein or the intermembrane space.

The inventors have further shown, that the RESA1 protein is a substrate of the MIA40 (Mitochondrial intermembrane space import and assembly protein 40 also designated Coiled-coil-helix-coiled-coil-helix domain-containing protein 4 and encoded by gene *CHCHD4*) translocase.

Thus, in a preferred embodiment, the at least one mutated gene according to the present invention is at least one gene that encodes a protein that is a MIA40 protein substrate. A "MIA40 protein substrate" according to the present invention is which import and/or assembly in mitochondria involve the MIA40 protein. The involvement of MIA40 in the import and/or assembly of a protein in question may for example be verified by a decrease in steady state levels of the protein after knock down of MIA40 via specific siRNA (Sakowska et al, 2015) or pulse-chase labelling followed by mitochondria isolation and protein purification via immunoprecipitation (Fischer et al 2013).

In another preferred embodiment of the present invention, the nuclear gene encodes a protein that is involved in mitochondrial biogenesis and/or function. A protein that is involved in mitochondrial biogenesis is a protein that is involved in establishing or maintaining, in full or in part, a mitochondrium as such or its regular structure, comprising all of its regular constituents with their regular activity in a cell. A defect in mitochondrial structure and/or the transport, localisation or assembly of other mitochondrial proteins in a mutant of the respective protein is considered to be evidence for the involvement of said protein in mitochondrial biogenesis. A defect in mitochondrial function, such as energy metabolism, especially respiratory chain activity, fatty acid synthesis and function of other known mitochondrial proteins in a mutant of the respective protein is considered to be evidence for the involvement of said protein in mitochondrial function. All mitochondrial proteins are considered to represent proteins that are involved in mitochondrial biogenesis and/or function.

As noted in the background section of this application, mutations in several genes encoding mitochondrial proteins were identified as cause for disorder. Within the context of the present invention these disorders are referred to as mitochondrial disorders or mitochondriopathies.

The following table discloses the respective proteins wherein such mutations have been observed, the related disease, the intramitochondrial localization of the protein, the phenotype of mutations with regard to protein levels, the MIM No. of the phenotype (Mendelian Inheritance in Man, https://www.omim.org), whether the respective protein is a MIA40 substrate, and the reference wherein the disease relation is described.

**Table 1: Protein/gene to disease relation between mitochondrial protein/gene and mitochondriopathies**

| No. | Protein | MIA40 Substrate | Localisation | Phenotye MIM no. | Phenotype | Disease | Reference |
|---|---|---|---|---|---|---|---|
| 1 | Augmenter Of Liver Regeneration; ALR | Yes | IMS | 613076 | Absence of protein | Myopathy, mitochondrial progressive, with congenital cataract, hearing loss, and developmental delay | Di Fonzo et al. (2009) |
| 2 | Coiled-Coil-Helix-Coiled-Coil-Helix Domain-Containing Protein 10; CHCHD10 | Yes | IMS | 616209 | | Myopathy, isolated mitochondrial, autosomal dominant Frontotemporal dementia and/or amyotrophic lateral sclerosis 2 Spinal muscular atrophy, Jokela type | Bannwarth et al. (2014) |
| | | | | 615911 | | | |
| | | | | 615048 | | | |
| | | | | | | | Muller et al. (2014) |
| | | | | | | | Johnson et al. (2014) |
| 3 | Cytochrome c oxidase, subunit 6B1 COX6B1 | Yes | IM | 220110 | Absence of protein | Mitochondrial Complex IV deficiency | Massa et al. (2008) |
| | | | | | | | Abdulhag et al. (2015) |
| 4 | Cytochrome C Oxidase Assembly Factor 6; COA6 | Yes | IMS | 616501 | Absence of protein | Cardioencephalomyopathy, fatal infantile, due to cytochrome c oxidase deficiency | Ghosh et al. (2014) |
| | | | | | | | Baertling et al. (2015) |
| 5 | Cytochrome C Oxidase Assembly Factor 5; COA5 | Yes | IMS | 616500 | Absence of protein | Cardioencephalomyopathy, fatal infantile, due to cytochrome c oxidase deficiency | Huigsloot et al. (2011) |
| 6 | Mitochondrial Calcium Uptake Protein 1; MICU1 | Yes | IMS | 615673 | Not known | Myopathy with extrapyramidal signs | Logan et al. (2014) |
| 7 | NADH -Ubiquinone Oxidoreductase 1 Alpha Subcomplex, 8; NDUFA8 | Predicted | IM | 252010 | Not known | Mitochondrial complex I deficiency | Bugiani et al. (2004) |
| 8 | NADH-Ubiquinone Oxidoreductase 1 Beta Subcomplex, 10; NDUFB10 | Yes | IM | - | Absent | Mitochondrial complex I deficiency | Friederich et al. (2017) |
| 9 | NADH-ubiquinone oxidoreductase fe-s protein 1; NDUFS1 | Predicted | IM | 252010 | Absence of protein | Mitochondrial complex I deficiency | Benit et al. (2001) |
| | | | | | | | Hoefs et al. (2010) |
| | | | | | | | Ferreira et al. (2011) |
| | | | | | | | Martin et al. (2005) |
| 10 | Translocase of inner mitochondrial membrane 8, TIMM8A | Yes | IMS | 304700 | Absence of protein | Mohr-Tranebjaerg syndrome | Tranebjaer g et al. (1997) |
| | | | | | | | Tranebjaer g et al. (2000) |
| 11 | Respiratory chain Assembly protein 1 RESA1 | Yes | IMS | 220110 | Absence of protein | Mitochondrial Complex IV deficiency | Martinez Lyons et al. (2016) |
| 12 | ADENYLATE KINASE 2; AK2 | No | IMS | 267500 | Absence of protein | Reticular dysgenesis | Lagresle-Peyrou et al. (2009) |
| 13 | Cytochrome C Oxidase, Subunit 8A; COX8A | No | IM | 220110 | Lower levels | Mitochondrial complex IV deficiency | Hallmann et al. (2016) |
| 14 | Cytochrome C Oxidase Assembly Factor; COX14 | No | IM | 220110 | Lower levels | Mitochondrial complex IV deficiency | Weraarpac hai et al. (2012) |
| 15 | NADH-Ubiquinone Oxidoreductase 1 Beta Subcomplex, 9; NDUFB9 | No | IM | 252010 | Lower levels | Mitochondrial complex I deficiency | Haack et al. (2012) |
| 16 | Cytochrome C Oxidase Assembly Factor; COX15 | No | IM | 615119 | Not known | Cardioencephalomyopathy, fatal infantile, due to cytochrome c oxidase deficiency 2 Leigh syndrome due to cytochrome c oxidase deficiency | Bugiani et al. (2005) |
| | | | | 256000 | | | Antonicka et al. (2003) |
| 17 | Cytochrome C Oxidase, Subunit 6A1; COX6A1 | No | IM | 616039 | Lower levels | Charcot-Marie-Tooth disease, recessive intermediate D | Tamiya et al. (2014) |
| 18 | DIABLO/SMAC | No | IMS | 614152 | Absence of protein | DEAFNESS, AUTOSOMAL DOMINANT 64; DFNA64 | Cheng et al. (2011) |
| 19 | FAD-Dependent Oxidoreductase Domain-Containing Protein 1; FOXRED1 | No | IM | 256000 | Not known (over expression of mutant rescued the functionality of Complex 1 | Leigh syndrome due to mitochondrial complex I deficiency | Formosa et al. (2015) |
| | | | | 252010 | | | |
| 20 | MIC13 | No | IM | - | Absence of prot plus absence of MIC10 | Mitochondrial hepato-encephalopathy | Zeharia et al. (2016) |
| 21 | NADH -Ubiquinone Oxidoreductase 1 Alpha Subcomplex, 12; NDUFA12 | No | IM | 256000 | Absent | Leigh syndrome due to mitochondrial complex 1 deficiency | Ostergaard et al. (2011) |
| 22 | NADH Dehydrogenase (Ubiquinone) Complex I, Assembly Factor 2; NDUFAF2 | No | IM | 256000 | Absence of protein | Leigh syndrome due to mitochondrial complex i deficiency | Ogilvie et al. (2005) |
| | | | | 252010 | | | Calvo et al. (2010) |
| 23 | NADH Dehydrogenase (Ubiquinone) Complex I, Assembly Factor 1; **NDUFAF1** | No | IM | 252010 | Lower levels | Mitochondrial complex I deficiency | Dunning et al. (2007) |
| | | | | | | | Fassone et al. (2011) |
| 24 | NADH Dehydrogenase (Ubiquinone) Complex I, Assembly Factor 4; **NDUFAF4** | No | IM | 252010 | Lower levels | Mitochondrial complex I deficiency | Saada et al. (2008) |
| 25 | NADH Dehydrogenase (Ubiquinone) Complex I, Assembly Factor 5; **NDUFAF5** | No | IM | 252010 | Lower levels | Mitochondrial complex I deficiency | Gerards et al. (2010) |
| | | | | | | | Saada et al. (2012) |
| 26 | NADH-Ubiquinone Oxidoreductase Fe-S Protein 4; **NDUFS4** | No | IM | 256000 | Absence of protein | Leigh syndrome due to mitochondrial complex i deficiency | van den Heuvel et al. (1998) |
| | | | | 252010 | | | Budde et al. (2000) |
| | | | | | | | Petruzzella et al. (2001) |
| | | | | | | | Anderson et al. (2008) |
| | | | | | | | Assereto et al. (2014) |
| 27 | NADH Dehydrogenase (Ubiquinone) Complex I, Assembly Factor 6; **NDUFAF6** | No | IM | 256000 | Lower levels | Leigh syndrome due to mitochondrial complex I deficiency | McKenzie et al. (2011) |
| | | | | 252010 | | | Pagliarini et al. (2008) |
| | | | | | | | Bianciardi et al. (2016) |
| | | | | | | | Kohda et al. (2016) |
| 28 | Succinate Dehydrogenase Complex, Subunit D, Integral Membrane Protein; **SDHD** | No | IM | 114900 | Not known | Carcinoid tumors, intestinal Cowden syndrome 3 Merkel cell carcinoma, somatic Mitochondrial complex II deficiency Paraganglioma and gastric stromal sarcoma Paragangliomas 1, with or without deafness | McWhinney et al. (2007) |
| | | | | 615106 | | | Kytola et al. (2002) |
| | | | | 252011 | | | |
| | | | | 606864 | | | Ni et al. (2008) |
| | | | | 168000 | | | |
| | | | | 171300 | | | Baysal et al. (2000) |
| | | | | | | | Astrom et al. (2003) |
| 29 | Sulfite oxidase , **SUOX** | No | IMS | 272300 | Not known | SULFITE OXIDASE DEFICIENCY | Seidahmed et al. (2005) |
| 30 | Tetratricopeptide Repeat Domain-Containing Protein 19; **TTC19** | No | IM | 615157 | Absence of protein | Mitochondrial complex III deficiency, nuclear type 2 | Nogueira et al. (2013) |

| No | Protein | MIA40 Substrate | Localisation | Phenotype MIM no. | Phenotype | Disease | Reference |
|---|---|---|---|---|---|---|---|
| 31. | Apoptogenic Protein 1, Mitochondrial; APOPT1 | No | IM | 220110 | Not known | Mitochondrial complex IV deficiency | Melchionda et al. (2014) |
| 32. | Atp Synthase, H+ Transporting, Mitochondrial F1 Complex, Alpha Subunit 1; ATP5A1 | No | IM | 616045 | Not known | Combined oxidative phosphorylation deficiency 22 Mitochondrial complex (ATP synthase) deficiency, nuclear type | Lieber et al. (2013) |
| | | | | 615228 | | | |
| 33. | Atp Synthase, H+ Transporting, Mitochondrial F1 Complex, Epsilon Subunit; ATP5E | No | IM | 614053 | Not known | Mitochondrial complex V (ATP synthase) deficiency, nuclear type 3 | Mayr et al. (2010) |
| 34. | Atp Synthase, Mitochondrial F1 Complex, Assembly Factor 2; ATPAF2 | No | IM | 604273 | Not known | Mitochondrial complex V (ATP synthase) deficiency, nuclear type 1 | De Meirleir et al. (2004) |
| 35. | Cytochrome C Oxidase Assembly Factor Cox10; COX10 | No | IM | 256000 | Not known | Leigh syndrome due to mitochondrial COX4 deficiency Mitochondrial complex IV | Coenen et al. (2004) |
| | | | | 220110 | | | |
| | | | | | | | Antonicka et al. |
| 36. | Cytochrome C Oxidase Assembly Factor; COX20 | No | IM | 220110 | Lower levels | Mitochondrial complex IV deficiency | Szklarczyk et al. (2013) |
| 37. | Cytochrome C Oxidase Assembly Protein; SCO1 | No | IM | 220110 | Not known | Mitochondrial complex IV deficiency | Stiburek et al. (2009) |
| | | | | | | | Valnot et al. (2000) |
| | | | | | | | Stiburek et |
| 38. | Cytochrome C1; CYC1 | No | IMS | 615453 | Lower levels | Mitochondrial complex III | Gaignard et al. (2013) |
| 39. | Cytochrome C Oxidase Assembly Factor; COX10 | No | IM | 256000 | Not known | Leigh syndrome due to mitochondrial COX4 deficiency Mitochondrial complex IV deficiency | Antonicka |
| | | | | 220110 | | | et al. (2003) |
| | | | | | | | Coenen et al. (2004) |
| 40. | Fast Kinase Domains 2; FASTKD2 | No | IM | 220110 | Lower levels | Mitochondrial complex IV deficiency | Ghezzi et al. (2008) |
| 41. | Lyr Motif-Containing Protein 7; LYRM7 | No | IM | 615838 | Lower levels | Mitochondrial complex III deficiency, nuclear type 8 | Dallabona et al. (2016) |
| 42. | NADH Dehydrogenase 1 Alpha | No | IM | 252010 | Not known | Mitochondrial complex I | Berger et al. |
| 43. | NADH-Ubiquinone Oxidoreductase 1 Alpha Subcomplex, 2; | No | IM | 256000 | Not known Assembly of complex I affected | Leigh syndrome due to mitochondrial complex I deficiency | Hoefs et al. (2008 ) |
| 44. | NADH-Ubiquinone Oxidoreductase 1 | No | IM | 256000 | Not known | Leigh syndrome due to mitochondrial complex I deficiency | van den Bosch |
| 45. | NADH-ubiquinone oxidoreductase fe-s protein 2; NDUFS | No | IM | 252010 | Not known Assembly of complex I affected | Mitochondrial complex I deficienc y | Ugalde et al. (2004) |
| | | | | | | | Loeffen et al. (2001) |
| 46. | NADH-Ubiquinone Oxidoreductase Flavoprotein 1: | No | IM | 252010 | Not known | Mitochondrial complex I deficienc y | Varghese et al. (2015) |
| 47. | NADH-Ubiquinone Oxidoreductase | No | IM | 252010 | Lower levels | Mitochondrial complex Ideficiency | Benit et al. (2004 |
| 48. | Nucleotide-Binding Protein-Like Protein; NUBPL | No | IM | 252010 | Not known | Mitochondrial complex I deficiency | Kevelam et al. (2013) |
| | | | | | | | Tucker et al (2012) |
| 49. | NADH-Ubiquinone Oxidoreductase Fe-S Protein 8; NDUFS8 | No | IM | 256000 | Not known | Leigh syndrome due to mitochondrial complex I deficiency | Loeffen et al. (1998) |
| | | | | | | | Procaccio and Wallace (200 |
| 50. | NADH -Ubiquinone Oxidoreductase 1 Beta Subcomplex, 3; NDUFB3 | No | IM | 252010 | Not known | Mitochondrial complex I deficiency | Calvo et al. (2012) |
| | | | | | | | Haack et al. (2012) |
| | | | | | | | l amont et |
| 51. | NADH-Ubiquinone Oxidoreductase 1 Alpha Subcomplex, 1; NDUFA1 | No | IM | 252010 | Lower levels | Mitochondrial complex I deficiency | Fernand ez-Moreira et al. (2007) |
| | | | | | | | Potluri et al. (2009) |
| | | | | | | | Mavr et |
| 52. | NADH Dehydrogenase (Ubiquinone) Complex I, Assembly Factor 3; NDUFAF3 | No | IM | 252010 | Not known Assembly of complex I affected | Mitochondrial complex I deficiency | Saada et al. (2009) |
| 53. | NADH -Ubiquinone Oxidoreductase Fe-S ProteiN 6; NDUFS6 | No | IM | 252010 | Not known | Mitochondrial complex I deficiency | Kirby et al. (2004) Spiegel et al. (2009) |
| 54. | NADH-Ubiquinone Oxidoreductase Fe-S Protein 3; NDUFS3 | No | IM | 256000 252010 | Not known | Leigh syndrome due to mitochondrial complex I deficiency | Benit et al. (2004) Haack et |
| 55. | NADH Dehydrogenase 1 Beta Subcomplex, 11; **NDUFB11** | No | IM | 252010 | Not known | Mitochondrial complex I deficiency Linear skin defects with multiple congenital anomalies 3 | Van Rahden et al. (2015) |
| | | | | 300952 | | | |
| | | | | | | | Kohda et al. (2016) |
| 56. | Succinate Dehydrogenase Complex, Subunit A, Flavoprotein; **SDHA** | No | IM | 613642 | Not known | Cardiomyopathy, dilated, 1GG Leigh syndrome; Mitochondrial respiratory chain complex II deficiency Paragangliomas 5 | Bourgeron et al. (1995) |
| | | | | 256000 | | | |
| | | | | 252011 | | | Parfait et al.(2000) |
| | | | | 614165 | | | |
| | | | | | | | Levitas et al. (2010) |
| | | | | | | | Burnichon et al. (2010) |
| 57. | Succinate Dehydrogenase Complex Assembly Factor 1; **SDHAF1** | No | IM | 252011 | Not known | Mitochondrial complex II deficiency | Ghezzi et al. (2009) |
| 58. | SURFEIT 1; **SURF1** | No | IM | 616684 | Not known | Charcot -Marie-Tooth disease, type 4K Leigh syndrome, due to COX | Pequignot et |
| | | | | 256000 | | | al. (2001) |
| | | | | | | | Poyau et al. (2000) |
| 59. | Transmembrane Protein 126B; **TMEM126B** | No | IM | 252010 | Not known | Mitochondrial complex I deficiency | Sanchez-Caballero et al. |
| | | | | | | | (2016) Alston et al. (2016) |
| 60. | Tetratricopeptide Repeat Domain-Containing Protein 19; **TTC19** | No | IM | 615157 | Absenc e of protein | Mitochondrial complex III deficiency, nuclear type 2 | Nogueira et al. (2013) |
| 61. | Translational Activator Of Mitochondrially Encoded Cytochrome C Oxidase Subunit I; **TACO1** | No | IM | 220110 | Not known | Mitochondrial complex IV deficiency | Makrythanasi s et al. (2014) |
| 62. | Translocase Of Inner Mitochondrial Membrane Domain-Containing Protein 1; TIMMDC1 | No | IM- C1 | 252010 | not known | Mitochondrial complex I deficienc y | Kremer et al. (2017) |
| 63. | Transmembrane Protein 70; TMEM70 | No | IM | 614052 | Not known | Mitochondrial complex V (ATP synthase) deficiency, nuclear type 2 | Spiegel et al. (2011) |
| | | | | | | | Cizkova et al. (2008) |
| 64. | Ubiquinol-Cytochrome C Reductase Complex Assembly Factor 2; UQCC2 | No | IM | 615824 | Not known | Mitochondrial complex III deficiency, nuclear type 7 | Tucker et al. (2013) |
| 65. | Ubiquinol-Cytochrome C Reductase Complex Assembly Factor 3; UQCC3 | No | IM | 616111 | Absence of protein | Mitochondrial complex III deficiency, nuclear type 9 | Wanschers et al. (2014) |
| 66. | Ubiquinol-Cytochrome C Reductase Core Protein li; UQCRC2 | No | IM | 615160 | Lower levels | Mitochondrial complex III deficiency, nuclear type 5 | Miyake et al. (2013) |
| | | | | | | | Gaignard et al. (2017) |
| 67. | Ubiquinol-Cytochrome C Reductase, Complex lii Subunit Vii, 9.5-Kd; UQCRQ | No | IM | 615157 | Not known | Mitochondrial complex III deficiency, nuclear type 4 | Barel et al. (2008) |
| 68. | Ubiquinol-Cytochrome C Reductase-Binding Protein; UQCRB | No | IM | 615157 | Not known | Mitochondrial complex III deficiency, nuclear type 3 | Haut et al. (2003) |

Based on the above disclosed protein/gene to disease relation, according to another preferred embodiment of the present invention, the nuclear gene encodes a protein selected from at least one protein selected form the group comprising the proteins RESA1, FOXRED1, augmenter of liver regeneration protein (ALR), coiled-coil-helix-coiled-coil-helix domain-containing protein 10 (CHCHD10), cytochrome c oxidase, subunit 6B1 (COX6B1), cytochrome c oxidase assembly factor 6 (COA6), cytochrome c oxidase assembly factor 5 (COA5), mitochondrial calcium uptake protein 1 (MICU1), NADH -ubiquinone oxidoreductase 1 alpha subcomplex 8 (NDUFA8), NADH-ubiquinone oxidoreductase 1 beta subcomplex 10 (NDUFB10), NADH-ubiquinone oxidoreductase FE-S protein 1 (NDUFS1) translocase of inner mitochondrial membrane 8 (TIMM8A), adenylate kinase 2 (AK2), cytochrome c oxidase subunit 8A (COX8A), cytochrome C oxidase assembly factor (COX14), NADH-ubiquinone oxidoreductase 1 beta subcomplex 9 (NDUFB9), cytochrome c oxidase assembly factor (COX15), cytochrome C oxidase subunit 6A1 (COX6A1), DIABLO/SMAC, FAD-dependent oxidoreductase domain-containing protein 1 (FOXRED1), MIC13, NADH -ubiquinone oxidoreductase 1 alpha subcomplex 12 (NDUFA12), NADH dehydrogenase (ubiquinone) complex I assembly factor 1 (NDUFAF1), NADH dehydrogenase (ubiquinone) complex I assembly factor 4 (NDUFAF4), NADH-ubiquinone oxidoreductase Fe-S protein 4 (NDUFS4), NADH dehydrogenase (ubiquinone) complex I assembly factor 6 (NDUFAF6) succinate dehydrogenase complex, subunit D, integral membrane protein; (SDHD), sulfite oxidase (SUOX), tetratricopeptide repeat domain-containing protein 19 (TTC19), mitochondrial apoptogenic protein 1 (APOPT1), H+ transporting ATP synthase mitochondrial F1 complex alpha subunit 1 (ATP5A1), H+ transporting ATP synthase mitochondrial F1 complex epsilon subunit (ATP5E), Atp Synthase, mitochondrial F1 complex assembly factor 2 (ATPAF2), cytochrome c oxidase assembly factor 10 (COX10), cytochrome c oxidase assembly factor 20 (COX20), cytochrome c oxidase assembly protein (SCO1), cytochrome C1 (CYC1), fast kinase domains 2 (FASTKD2), Lyr motif-containing protein 7 (LYRM7), NADH dehydrogenase 1 alpha subcomplex 11 (NDUFA11), NADH-ubiquinone oxidoreductase 1 alpha subcomplex 2 (NDUFA2), NADH-ubiquinone oxidoreductase 1 alpha subcomplex 9 (NDUFA9), NADH-ubiquinone oxidoreductase Fe-S protein 2 (NDUFS2), NADH-ubiquinone oxidoreductase flavoprotein 1 (NDUFV1), nucleotide-binding protein-like protein (NUBPL), NADH-ubiquinone oxidoreductase Fe-S protein 8 (NDUFS8), NADH-ubiquinone oxidoreductase beta subcomplex 3 (NDUFB3), NADH-ubiquinone oxidoreductase 1 alpha subcomplex 1 (NDUFA1), NADH dehydrogenase (ubiquinone) Complex I assembly factor 3 (NDUFAF3), NADH-ubiquinone oxidoreductase Fe-S protein 6 (NDUFS6), NADH dehydrogenase 1 beta subcomplex 11 (NDUFB11), succinate dehydrogenase complex subunit a flavoprotein (SDHA), succinate dehydrogenase complex assembly factor 1 (SDHAF1), SURFEIT 1 (SURF1), transmembrane protein 126B (TMEM126B), tetratricopeptide repeat domain-containing protein 19 (TTC19), translational activator of mitochondrially encoded cytochrome c oxidase subunit I (TACO1), translocase of inner mitochondrial membrane domain-containing protein 1 (TIMMDC1), transmembrane protein 70 (TMEM70), ubiquinol-cytochrome c reductase complex assembly factor 2 (UQCC2), ubiquinol-cytochrome c reductase complex assembly factor 3 (UQCC3), ubiquinol-cytochrome c reductase core protein II (UQCRC2), ubiquinol-cytochrome c reductase complex III subunit VII (UQCRQ), and/or ubiquinol-cytochrome c reductase-binding protein (UQCRB). More preferably the nuclear gene encodes FOXRED1. In the most preferred embodiment, the gene encodes RESA1 RESA1 (Respiratory Chain Assembly1, also termed COA7 or C1orf163).

In a further preferred embodiment, the mutation according to the present invention results in the substitution, deletion, and/or insertion of at least one amino acid in a mitochondrial protein. Generally a mutation in the primary sequence of protein may result in different effects on the secondary or tertiary structure of a protein. According to the present invention the mutation preferably results in at least a partial misfolding or instability of the protein. A misfolding may be the deviation of the tertiary structure of the whole protein or at least parts of the protein, such as a domain or subunit, from the wildtype form of the protein. A mutation might also result in a changed assembly of the mutation protein into a protein complex or into a membrane in comparison to the wildtype protein. The misfolding or instability might further lead to a mistargeting of the protein within mitochondria, a decreased half-life of the protein in a cell, and/or an increased susceptibility of mutated protein to cellular proteases, especially the proteases of the proteasome. The misfolding of a mutated protein may also be verified by a changed X-ray structure, NMR (nuclear magnetic resonance) spectra or CD (circular dichroism) spectrum in comparison to the respective wild type protein.

In a highly preferred embodiment, the mutation is a mutation causing a substitution of amino acid 137 or a deletion of the 47 amino acids encoded by exon 2 of RESA1.

The inhibitor of proteolytic activity according to the present invention preferably inhibits the 26S and/or 19S proteasome. Thus, the inhibitor of proteolytic activity may preferably be a ubiquitin ligase inhibitor, a ubiquitin specific peptidase inhibitor, a ubiquitin-like modifier activating enzyme inhibitor, and/or a ubiquitin-specific protease inhibitor. In a more specific embodiment, the inhibitor of proteolytic activity may inhibit chymotrypsin-like and/or peptidylglutamyl peptide hydrolysing activity.

In a specific embodiment of the invention the inhibitor of proteolytic activity may be a molecules under development or any antibody and may selected from group consisting of MG-132, bortezomib (Velcade®, Millennium Pharmaceuticals), carfilzomib (Kyprolis®, developed by Onyx Pharmaceuticals), CEP28331, Proteasome inhibitor ONX-0914, VL01, E18770, Ixazomib citrate (MLN9708), NPI-0052(salinosporamide A), HCV proteasome inhibitor, HIV Proteasome Inhibitor, KRX-040 (perifosine), MLN-273, MLN-519 (formerly known as LDP-519), tetra- acridines, MLN4924 (inhibitor of NEDD8), HBX19818, HBX41108 (USP7, protease 7 Inhibitors), ubiquitin ligases, USP8 Inhibitors (cysteine-proteases), CC12507, HBX99200, PI- 083, MLN 9708, MLN 4924, MLN 519, ONX 0912, CEP-1877, NPI-0047, NPI- 0052, BU-32 (NSC D750499-S), PR-171, IPSI-001, and natural products with proteasome- inhibitory effects, such as green tea polyphenol (-)-epigallocatechin-3-gallate (EGCG), soy isoflavone genistein, and the spice turmeric compound curcumin.

More preferably the inhibitor of proteolytic activity according to the present invention is selected from the group comprising inhibitors of proteolytic activity, especially proteasome inhibitors, from the boronate compound family, such as bortezomib (Velcade, Millennium Pharmaceuticals), from the epoxyketone family of proteasome inhibitors such as carfilzomib (Kyprolis®, developed by Onyx Pharmaceuticals), peptide boronic acid compounds, such as ixazomib (Millenium Pharmaceuticals), the structural family of beta Lactones, such as marizomib or the synthetic peptide MG132. Most preferably, the inhibitor of proteolytic activity is selected from bortezomib, carfilzomib or MG132. Generally several protease inhibitors may be used in combination according to the present invention.

In a preferred embodiment, the inhibitor of proteolytic activity may be a polynucleotide, preferably an RNA molecule that reduces directly or indirectly the proteolytic activity within the cell. Most preferably the RNA molecule is a small inhibitory RNA molecule. The inhibitor may also be a DNA that encodes a compound that otherwise reduces directly or indirectly the proteolytic activity within the cell. For example, the DNA may encode a small inhibitory RNA molecule, or other elements that regulate gene expression. The polynucleotide may be in form of a vector, for example a DNA or RNA viral vector, or in form of a plasmid.

Alternatively, the inhibitor of proteolytic activity may be a polypeptide, especially a polypeptide that reduces directly or indirectly the proteolytic activity within a cell. The polypeptide may for example be regulatory protein, such as a regulatory protein that directly inhibits the activity of a proteolytic protein or decreases the expression of genes. The polypeptide may also preferably an antibody, or an antibody derived molecule such as a fragment of an antibody, for example a Fab-fragment, or a diabody.

Preferably, proteolytic activity in the above embodiment is the proteolytic activity of the proteasome. Thus, preferably a proteolytic activity within the cytosol is inhibited or reduced.

In a further embodiment of the present invention, the mitochondriopathy is characterized by a decreased amount of at least one mitochondrial protein as described herein. Within this embodiment, the amount of said protein in the mitochondria of a subject suffering from a mitochondriopathy, thus in a subject wherein the gene encoding the respective protein carries a mutation, is reduced in comparison to the mitochondria in a healthy subject, thus a subject wherein the gene encoding the respective protein does not carry a mutation. The reduction may be determined by quantifying the amount of the respective protein in relation to the total mitochondrial protein amount. Methods for determining protein amounts are generally known in the art, including western blotting and analysis with an antibody against the respective protein. A respective determination of relative protein amounts is described in the experiments of this application.

The mitochondriopathy according to the present invention may be selected from mitochondrial myopathy, preferably progressive mitochondrial myopathy, cardiomyopathy, preferably dilated 1GG; mitochondrial myopathy with congenital cataract, hearing loss, and developmental delay, isolated autosomal dominant mitochondrial myopathy, mitochondrial myopathy with extrapyramidal signs, cardioencephalomyopathy, preferably fatal infantile cardioencephalomyopathy, frontotemporal dementia and/or amyotrophic lateral sclerosis 2, spinal muscular atrophy, preferably Jokela type spinal muscular atrophy, myopathy with extrapyramidal signs, mitochondrial complex I deficiency, mitochondrial complex IV deficiency, Mohr-Tranebjaerg syndrome, reticular dysgenesis, mitochondrial complex IV deficiency, Leigh syndrome due to cytochrome c oxidase deficiency, mitochondrial hepatoencephalopathy, Cowden syndrome 3, somatic merkel cell carcinoma, mitochondrial complex II deficiency, pagangliomas 1, preferably with or without deafness; paragangliomas 5, sulfate oxidase deficiency, mitochondrial complex III deficiency, preferably nuclear type 2; combined oxidative phosphorylation deficiency 22, Mitochondrial complex V deficiency, preferably nuclear type 1, 3 or 4; mitochondrial complex I deficiency, Leigh syndrome due to mitochondrial complex I deficiency, Leigh syndrome due to COX IV deficiency, and/or mitochondrial complex III deficiency, preferably nuclear type 2, 3, 4, 5, 7, 8, or 9.

Most preferably, the mitochondriopathy is a leukoencephalopathies.

According to the present invention, the mitochondriopathy is not caused by a mutation in a mitochondrial DNA. In a specific embodiment, the mitochondriopathies according to the present invention do not include a mitochondriopathy selected from at least Kearns-Sayre syndrome, mitochondrial encephalomyopathy-lactic-acídosís-stroke like episodes (MELAS), myoclonic epilepsy and ragged-red-fibers (MERRF), Leber hereditary optic neuropathy (LHON), Leigh's syndrome, neuropathy-ataxia-retinitis pigmentosa (NARP) and progressive external opthalmoplegia (PEO).

In an alternative aspect, the invention relates to an inhibitor of proteolytic activity for use in a method of preventing a mitochondriopathy in a subject who is at risk of developing a mitochondriopathy due to a genetic mutation. Within the context of the present invention, a person who is at risk of developing a mitochondriopathy is a subject who has been diagnosed with a at least one mutation in a gene encoding at least one of the above described protein, but does not show any clinical signs of a mitochondriopathy yet.

The subject in accordance with the present invention is preferably a human subject, especially a human patient.

The protease inhibitor according to the present invention is used, i.e. administered to a subject, in an effective dose. The effective dose and a suitable dosage regime may be determined by the attending physician based on clinical factors. The effective dosage employed may vary depending on the particular compound employed, the mode of administration, the condition being treated and the severity of the condition being treated.

Any suitable route of administration may be employed for administering the inhibitor of proteolytic activity of the present invention. The inhibitor of proteolytic activity may be administered systemically or locally. For example, oral, rectal, topical, parenteral, such as for example intravenous, ocular, pulmonary or nasal administration may be employed. Preferably, the protease inhibitor according to the present invention is administered orally or intravenously.

The protease inhibitor according to the present invention may be administered as the sole active agent for the treatment of the above described conditions or may be administered in combination with a further agent active and/or supportive for the treatment of the conditions.

In a preferred embodiment, the inhibitor of proteolytic activity according to the present invention may be comprised in a composition or medicament that encompasses at least one pharmaceutically acceptable carrier and/or excipient. The pharmaceutically acceptable carrier and/or excipient useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the inhibitor of proteolytic activitys herein disclosed. In general, the nature of the carrier or excipients will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol, citric acid or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

In a further aspect, the present invention relates to the use an inhibitor of proteolytic activity as described above for treating a mitochondriopathy as described above.

Furthermore, the present invention relates to an inhibitor of proteolytic activity as described above for manufacturing a medicament for treating a mitochondriopathy as described above.

In still a further embodiment, the present invention relates to a method for treating a patient suffering from a mitochondriopathy caused by a genetic mutation as described herein or for preventing a mitochondriopathy caused by a genetic mutation.

In accordance with the embodiments described above, the invention generally also relates to a method for treating or preventing a mitochondriopathy caused by a genetic mutation by decreasing proteolytic activity within a cell. Generally, the specific embodiments of the invention as disclosed above may also be specific embodiments within this method for treating or preventing a mitochondriopathy caused by a genetic mutation by decreasing proteolytic activity within a cell.
The proteolytic activity may be decreased by using any mechanism that finally results in a decrease of proteolytic activity within a cell.

Thus, method comprises a step that decrease the proteolytic activity within a cell.

The step may decrease directly or indirectly the activity of a protease within the cell. Thus, the may reduce or abolish the transcription or translation of a gene encoding a protease or a protein that otherwise contributes to, or regulates the activity of proteolytic activity within a cell. The proteolytic activity may also be decreased by decreasing the activity of regulatory elements that themselves increase proteolytic activity, or by increasing the activity of regulatory elements that themselves decrease the activity of proteolytic activity in a cell.

The step of decreasing the proteolytic activity may comprise subjecting a cell to an inhibitor of proteolytic activity as described herein. The step of subjecting a cell to an inhibitor of proteolytic activity is preferably performed by administering said inhibitor to a human subject as disclosed above.

The step of decreasing the proteolytic activity may comprise a step of altering the genetic information within a cell. This step may for example comprise the disruption of at least one gene that contribute to the proteolytic activity within the cell to abolish the expression of said gene, the mutation of said gene, or the disruption or mutation of regulatory elements. Alternatively, the alteration of the genetic information may introduce genetic information that promotes the decrease of proteolytic activity by expressing inhibitory elements, for example proteins that decrease indirectly or directly decrease proteolytic activity within a cell. Various methods for altering the genetic information within a cell are known in the art.

The present invention shall be explained in more detail by the following figures and examples.

### Figures

Figure 1 shows the lower expression levels of RESA1 pathogenic mutants.
Figure 2 shows that RESA1 mutants degrade faster than wild type protein
Figure 3 shows the defective import of RESA1 mutants
Figure 4 shows the mislocalization of RESA1- exon2^{Δ} mutant (T - Total fraction; C - Cytosol fraction; M - Mitochondrial fraction)
Figure 5 shows the rescue of RESA1 mutants in HEK293 cells upon MG132 treatment
Figure 6 shows the increased mitochondrial localization of RESA1 mutants upon MG132 treatment.
Figure 7 shows the inhibition of proteasome rescues levels of RESA1 in patient cells.
Figure 8 shows the increased mitochondrial localization of RESA1 mutants in patient cell line upon proteasome inhibition
Figure 9 shows the experimental scheme for degradation experiment with Cycloheximide (CHX) (Fig. 9A) and with Cycloheximide (CHX) & MG132 (Fig. 9B).

### Examples

### 1. Characterization of RESA1 mutants

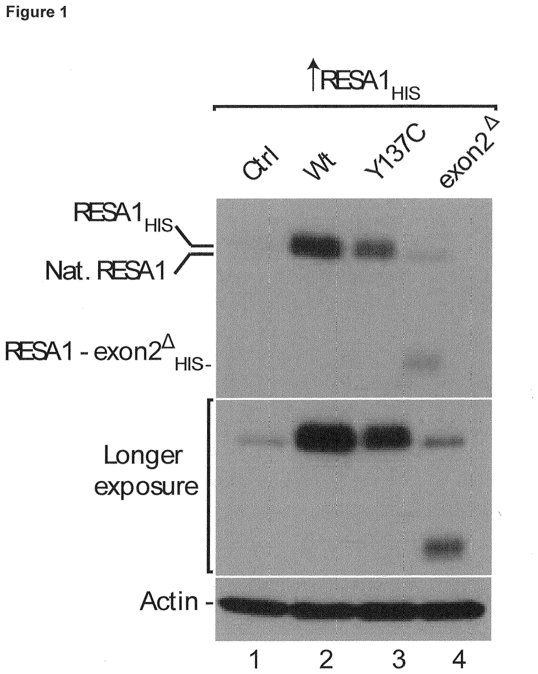

A RESA1 with single amino acid mutation (RESA1-Y137C) and RESA1 with a deletion of exon 2 (RESA1- exon2^{Δ}) (Martinez Lyons et al., 2016) were expressed in human HEK293 cells. HEK293 cells were transfected with plasmid encoding RESA1 (wild-type and mutants as indicated). After 24 h, cellular proteins were isolated and analyzed by reducing SDS-PAGE and western blotting. The results are shown in Figure 1.

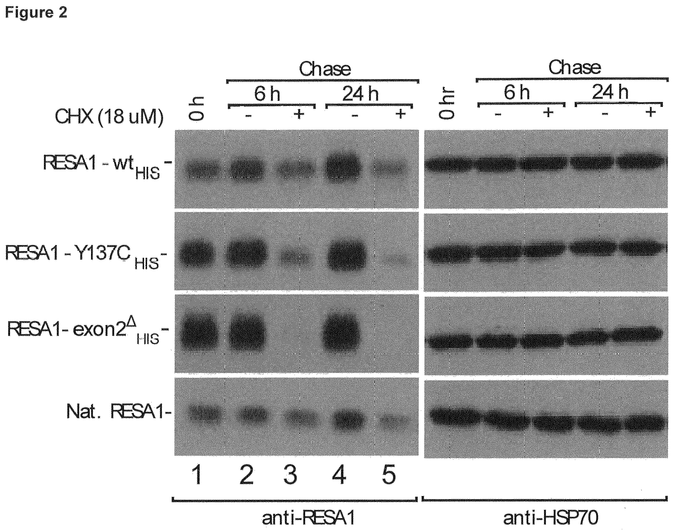

In a further experiment HEK293 cells were transfected with plasmid encoding RESA1 (wild-type and mutants as indicated). After 24 h, the cells were treated with cycloheximide (CHX) or vehicle control for specific time points. The results are shown in Figure 2.

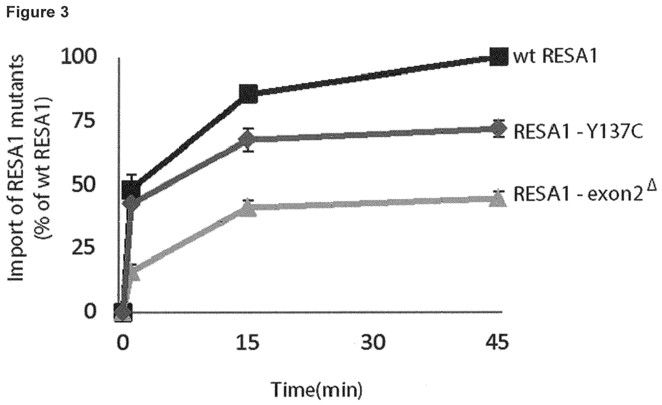

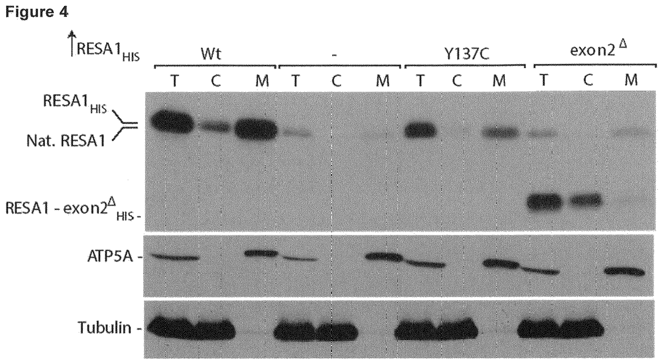

Significantly lower levels of mutant proteins were detected when compared to the wild type, which is in agreement with the absence of protein in patient fibroblasts (Martinez Lyons et al., 2016)(Fig. 1). The degradation kinetics of mutants by cycloheximide (protein synthesis inhibitor)/chase experiment were investigated. It was found that the mutants degrade faster than the wild type (Fig. 2).
Since the mutants were degrading faster, the import efficiency and localization of mutants in HEK293 cells was investigated. Wild-type and mutant radiolabeled [³⁵S] RESA1 precursors were imported into mitochondria isolated from HEK293 cells. The level of import of wtRESA1 after 45 min was set to 100%. The results of three biological experiments were analyzed and quantified. Means from three independent experiments are shown. Error bars indicate standard errors of the means. Samples were analyzed by reducing SDS-PAGE and autoradiography. Among the two mutants, RESA1- exon2^{Δ} displayed significant defect in the mitochondrial import (almost 70 %) (Fig. 3) contributing to its cytosolic localization in the cell (Fig. 4).

To analyze intramitochondrial localization, fractionation was carried out on HEK293 cells transfected with plasmid encoding RESA1 (wild-type and mutants). The results are shown in Figure 4. It can be concluded that defective mitochondrial import and cytosolic localization contributed to the faster degradation of RESA1 mutants.

The experiments were carried out according the materials and methods described below.

### 2. Proteasome involvement in degradation of RESA1 mutants

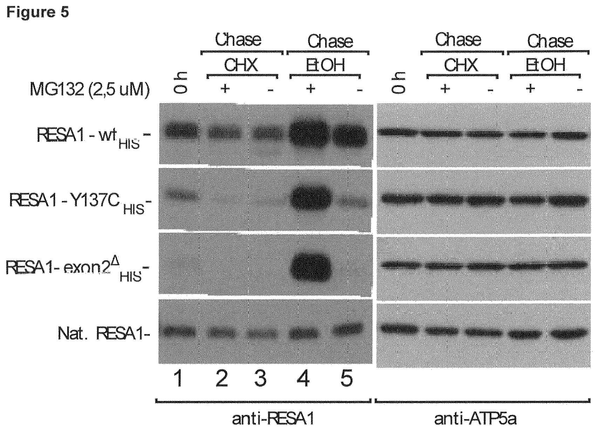

Since UPS is the main protein quality control machinery in cytosol, the role of proteasome in the degradation of these mutants by MG132 treatment (proteasome inhibitor) was tested. After 24 h of wild type and mutant RESA1 expression in HEK293 cells, cells were treated with either cycloheximide (CHX) or MG132 or both at specified time points. The results are shown in Figure 5. the results show that the degradation of RESA1 mutants can be rescued by proteasome inhibition under active translation, indicating that the newly synthesized or cytosol-localized mutants are sensitive to proteasome degradation

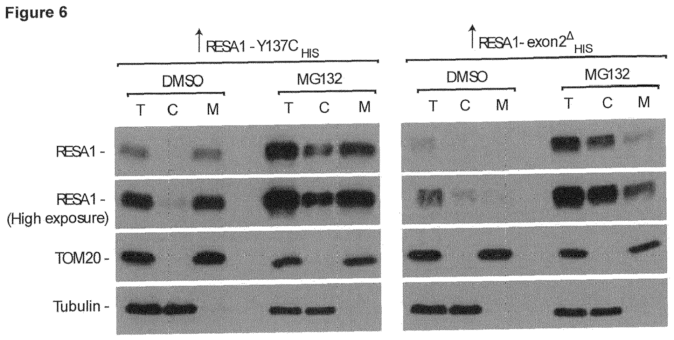

In a further experiment it was investigated whether the stabilization of mutant proteins in the cytosol increases their translocation to mitochondria In a furtjer experiment, after 24 h of wild type and mutant RESA1 expression, cells were treated with MG132 (2.5 uM) for 24 h and subjected to fractionation. The results are shown in Figure 6. The results show that in the presence of MG132, levels of RESA1 mutants were increased both in cytosol and mitochondria.

The experiments were carried out according the materials and methods described below.

### 3. Rescue of RESA1 mutants in patient cell line upon proteasome inhibition

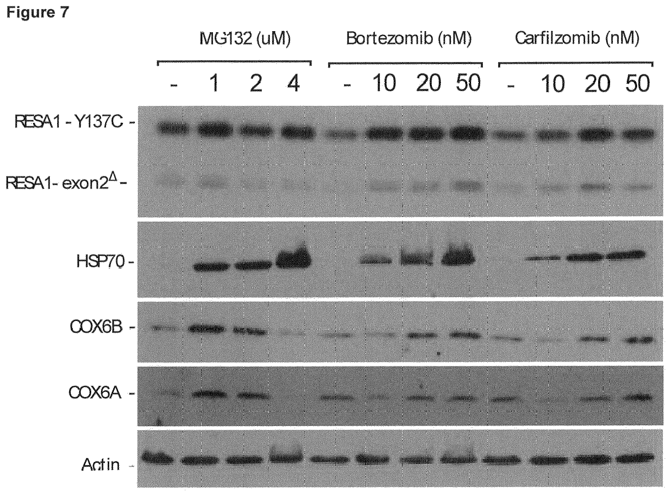

It was previously demonstrated the involvement of UPS in degradation of RESA1 mutants, when over expressed in HEK293 cells. To substantiate this mechanism the proteasome system in immortalized patient skin fibroblasts were treated with MG132, Bortezomib and Carfilzomib for 12 h. The samples were analyzed by reducing SDS-PAGE and western blotting. By proteasome inhibition, both the mutants of RESA1 (RESA1 - Y137C & RESA1-exon2^{Δ}) in patient cell line were recovered. The results are shown in Figure 7. Interestingly, an increase in the levels of proteins like COX6B and COX6A, components of Complex IV can be observed. This stabilization of mutants upon proteasome inhibition compliments the above findings in HEK293 cells.

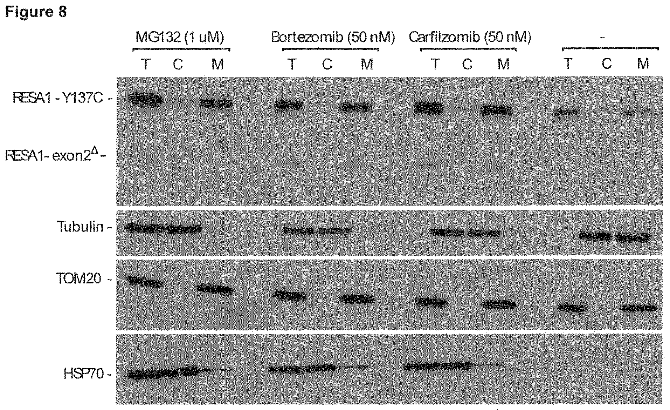

In a further experiment, the mitochondrial localization of the rescued mutants upon proteasome inhibition tested. Immortalized patient skin fibroblasts were treated with MG132, Bortezomib and Carfilzomib for 12 h and fractionation was carried out and the samples were analyzed by reducing SDS-PAGE and western blotting. A higher mitochondrial localization of rescued mutants in patient cell line can be observed (Figure 8). These results substantiate the involvement of UPS in degradation of mislocalised mitochondrial proteins.

The experiments were carried out according the materials and methods described below.

### 4. Material and Methods

### 4.1. Growth condition of HEK293 and fibroblasts cells:

HEK293 cells were cultured at 37°C with 5% CO₂ in standard DMEM containing high glucose (4.5g/L), supplemented with 10% (v/v) heat inactivated fetal bovine serum, 2 mM glutamine, 100 U/ml penicillin and 10 µg/ml streptomycin sulfate. For experiments, cells were grown in Dulbecco's modified Eagle medium (DMEM) containing low glucose (1.1g/L) or galactose (1.8 g/L) as and when described.

Fibroblast cells were cultured at 37°C with 5% CO₂ in standard DMEM containing high glucose (4.5g/L), supplemented with 10% (v/v) heat inactivated fetal bovine serum, 2 mM glutamine, 100 U/ml penicillin and 10 µg/ml streptomycin sulfate, 5 mM sodium pyruvate and 50 µg/ml uridine. For experiments, cells were grown in Dulbecco's modified Eagle medium (DMEM) containing low glucose (1.1 g/L) or galactose (1.8 g/L) as and when described. Mt4229i - Immortalized skin fibroblast from patient (19-year-old woman, first child of healthy unrelated parents) with RESA1 mutation

### 4.2. Immortalized skin fibroblast from a healthy donor:

The immortalized skin fibroblast were received from Prof. Massimo Zeviani laboratory, Mitochondrial Biology Unit, MRC, Cambridge.

### 4.3. Protein cellular extract:

HEK293 cells were grown in DMEM-low glucose medium for 24 h, and then cells were transferred to galactose medium and transfected with pcDNA3.1(+) vector encoding RESA_{HIS} (wild type and mutant) using Gene Juice Transfection Reagent (70967; Merck Millipore) according to manufacturer's protocol. After 24 h of expression, the cells were harvested by cell scrapper and washed twice with 1X PBS (without Ca²⁺ and Mg²⁺) by centrifugation (1000 x g, 5 min, 4 °C). The cell pellet was solubilized in RIPA buffer containing PMSF (2mM) for 30 min at 4 °C. The cell lysate was clarified by centrifugation (14,000 x g, 30min, 4 °C) and supernatant was collected. A small fraction was used to determine the protein concentration by bicinchoninic acid (BCA) protein assay. The remaining fraction was solubilized in Laemmli sample buffer containing 50 mM DTT and analyzed by reducing SDS-PAGE and western blotting.

RIPA buffer: 65 mM Tris base, 150 mM NaCl, 1% v/v NP-40, 0.25 % Sodium, Deoxycholate, 1 mM EDTA and 2mM PMSF, pH 7.4

### 4.4. Cycloheximide/Chase experiment:

HEK293 cells were grown in DMEM-low glucose medium for 24 h, and then cells were transferred to galactose medium and transfected with pcDNA3.1(+) vector encoding RESA_{HIS} using Gene Juice Transfection Reagent (70967; Merck Millipore) according to manufacturer's protocol. After 24 h of gene expression, the cells treated with either cycloheximide (CHX) or MG132 or both for specified time periods. Cellular protein extract collected at these time points were analyzed by reducing SDS-PAGE and western blotting.

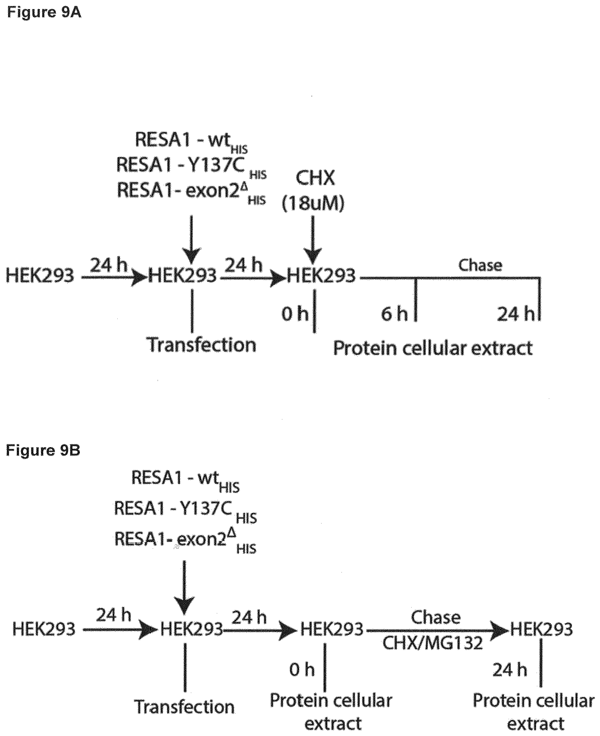

The experimental scheme for degradation experiment with Cycloheximide (CHX) is shown in Figure 9A. The experimental scheme for degradation experiment with Cycloheximide (CHX) & MG132 is shown in Figure 9B.

### 4.5. In organello import assay:

### 4.5.1. Synthesis of radiolabeled precursor for in vitro import:

The sequence coding for precursors (wild type and mutant RESA1) was cloned into separate pTNT vectors under an SP6 promoter. The recombinant plasmid was used to synthesize S³⁵ radiolabeled precursors by TNT SP6 Quick Coupled Transcription/Translation system (Promega) according to manufacturer's protocol. All the precursors were denatured in urea buffer and reduced with DTT before import.

**Table 2: Reagents for radioactive precursor synthesis**

| **Reagent** | **Amount** |
|---|---|
| TNT Quick Master MIX | 1x |
| Plasmid | 0.5 µg |
| S³⁵ Methionine | 1 µl |
| Nuclease free water | Make up to 25 µL |

### 4.5.2. Reduction of precursors:

The precursors were precipitated by adding twice the volume of ice-cold saturated ammonium sulphate solution and incubating on ice for 20 min. After precipitation, precursors were pelleted by centrifugation (20,000 x g, 10 min, 4° C) and resuspended in urea buffer containing DTT (50 mM) and incubated at RT for 15 min. The solution was centrifuged again and supernatant containing radiolabeled precursors was collected, aliquoted in to smaller volumes and snap frozen in liquid nitrogen and further stored in 30°C for later use. Urea buffer: 6M urea; 60 mM MOPS-KOH pH7.2

### 4.6. In vitro import assay

Only freshly isolated mitochondria were used for import experiments. The mitochondria were isolated by trehalose buffer method and concentration was adjusted to 10µg/µl. Radiolabeled precursor was incubated in an eppendorf tube containing import buffer supplemented with 5 nM ATP for 2 min at 30°C. After incubation, the import was initiated by adding mitochondria (30 µg) and aliquots were taken at specified time points. The aliquots were transferred to tubes containing IAA placed on ice to stop the import. As a control, precursors were precincubated with IAA to block the free cysteine residues, which thereby affects the import efficiency. After import, non-imported precursors were degraded by proteinase K treatment for 10 min at 4 °C. Then, proteinase K was inactivated by PMSF (2 mM) treatment for 5 min and samples were subjected to centrifugation (20,000 x g, 10 min, 4° C). The mitochondrial pellet was washed again by resuspending in high sucrose buffer containing 2 mM PMSF and centrifuged again. Finally, the mitochondria containing only imported radioactive precursor proteins were solubilized in Laemmli sample buffer with DTT (50 mM) and PMSF (4mM) and denatured at 65°C for 15 min. Samples were analyzed by reducing SDS-PAGE and autoradiography. The protein import was quantified by densitometry of the autoradiography images using the ImageQuantTL program. The level of import into control mitochondria at the indicated time point was set to 100%. The results of three biological experiments were analyzed, quantified, and the mean from three independent experiments are shown. Error bars indicate standard errors of the means.

Import buffer250mM sucrose; 80mM potassium acetate; 5mM magnesium acetate; 5mM methionine; 10mM sodium succinate; 20mM Hepes/KOH pH 7.4
High sucrose buffer : 500mM sucrose; 20mM Hepes/KOH pH 7.4

### 4.7. Fractionation experiment

### 4.7.1. Mitochondria isolation - Trehalose buffer method (For HEK293 cells)

Two dishes (150 cm²) containing 8 x 10⁶ cells were grown in DMEM - low glucose medium for 24 h and the medium was exchanged to galactose medium for the next 24h. The cells were harvested by cell scraper and washed twice with 1X PBS (without Ca²⁺ and Mg²⁺) by centrifugation (1000 x g, 5 min, 4 °C). The cell pellet was resuspended in ice-cold trehalose buffer containing BSA (2mg/ml) and PMSF (2 mM). The cell suspension was gently homogenized for 15 times with Dounce glass homogenizer, Sartorius (Catalogue no. BBI-8540705). The homogenate was subjected to centrifugation (1000 x g, 10 min, 4 °C) to pellet the cell debris. The supernatant containing mitochondria was carefully aspirated and centrifuged again. Then, supernatant was subjected to high speed centrifugation (10,000 x g, 10 min, 4 °C) to pellet the mitochondria. The pellet was resuspended in trehalose buffer (without BSA) and quantified by Bradford assay. Mitochondria were resuspended in 2 x Laemmli buffer with 50 mM DTT or 50 mM IAA and denatured at 65°C for 15 min. The samples were separated on SDS-PAGE and analyzed by Western blotting.
Trehalose buffer 300 mM trehalose; 10 mM HEPES; 10 mM KCI; 1 mM EDTA; 1 mM EGTA, pH 7.7

For fractionation, the supernatant obtained after homogenization was divided into three equal aliquots. One aliquot is saved as the total fraction, and other two aliquots were centrifuged at 10,000 x g for 10 min at 4 °C to collect the mitochondrial and cytosolic fractions. The proteins from total and cytosolic fraction were precipitated by pyrogallol red precipitation. Finally, all three fractions were resuspended in urea sample buffer with DTT (50 mM) for SDS-PAGE and western blotting analysis.

### 4.7.2. Mitochondria isolation - Limited swelling method (For fibroblast cell lines)

Two dishes (150 cm²) containing 8 x 10⁶ cells were grown in DMEM - low glucose medium for 24 h and the medium was exchanged to galactose medium for the next 24h. The cells were harvested by cell scraper and washed twice with 1X PBS (without Ca²⁺ and Mg²⁺) by centrifugation (1000 x g, 5 min, 4 °C). The cell pellet was resuspended in ice cold isotonic buffer containing 2mg/ml BSA and 2mM PMSF and subjected to centrifugation (1000 x g, 5 min, 4 °C). Then cell pellet was resuspended in cold Hypotonic Buffer (1 ml per 5 mg of cell pellet) and incubate on ice 5-7 min. The cell suspension was gently homogenized for 15 times with Dounce glass homogenizer, Sartorius (Catalogue no. BBI-8540705). Immediately after homogenization, cold Hypertonic Buffer was added to cell homogenate (1,1ml/1g cells). Then, volume of the homogenate was doubled up with ice cold Isotonic Buffer and then homogenate was subjected to centrifugation (1000 x g, 10 min, 4 °C) to pellet the cell debris. The supernatant containing mitochondria was carefully aspirated and centrifuged again. Then, supernatant was subjected to high speed centrifugation (10,000 x g, 10 min, 4 °C) to pellet the mitochondria. The pellet was resuspended in Isotonic Buffer (without BSA) and quantified by Bradford assay. Mitochondria were resuspended in 2 x Laemmli buffer with 50 mM DTT or 50 mM IAA and denatured at 65°C for 15 min. The samples were separated on SDS-PAGE and analyzed by Western blotting.
Isotonic buffer: 75 mM Mannitol, 225 mM Sucrose, 10 mM MOPS, 1 mM EGTA, pH 7.2,
Hypotonic Buffer : 100 mM Sucrose, 10 mM MOPS, 1 mM EGTA, pH 7.2
Hypertonic Buffer: 1.25 M Sucrose, 10 mM MOPS, pH 7.2

### 4.8. Inhibition of Proteasome in Patient fibroblasts

The patient fibroblast cells were grown in DMEM-low glucose medium for 24 h, and then cells were transferred to galactose medium containing different proteasome inhibitors (MG132, Bortezomib (PS-341, Cat. No. F1200, UBPbio), Carfilzomib (PR-171, Cat. No. S2853, Selleckchem). The treatment was carried out for 24 h and then protein cellular extract or fractionation was carried out as specified.

### REFERENCES

Calvo, S. E & Mootha, V. K. The mitochondrial proteome and human disease. Annu. Rev. Genomics Hum. Genet. 11, 25-44 (2010)
Koopman, W. J., Willems, P. H. & Smeitink, J. A. Monogenic mitochondrial disorders. N. EngL J. Med. 366, 1132-1141 (2012).
Suomalainen, A. & Battersby, BJ. Mitochondrial diseases: the contribution of organelle stress responses to pathology. Nat Rev Mol Cell Biol. 19(2):77-9 (2018)
Kerr, DS. Treatment of mitochondrial electron transport chain disorders: a review of clinical trials over the past decade. Mol Genet Metab.99(3), 246-55 (2010)
Wong, LJ. Mitochondrial syndromes with leukoencephalopathies. Semin Neurol. 32(1):55-61 (2012)
Di Fonzo A, Ronchi D, Lodi T, Fassone E, Tigano M, Lamperti C, Corti S, Bordoni A, Fortunato F, Nizzardo M, Napoli L, Donadoni C, Salani S, Saladino F, Moggio M, Bresolin N, Ferrero I, Comi GP. The mitochondrial disulfide relay system protein GFER is mutated in autosomal-recessive myopathy with cataract and combined respiratory-chain deficiency. Am J Hum Genet. 2009 May;84(5):594-604
Bannwarth S, Ait-EI-Mkadem S, Chaussenot A, Genin EC, Lacas-Gervais S, Fragaki K, Berg-Alonso L, Kageyama Y, Serre V, Moore DG, Verschueren A, Rouzier C, Le Ber I, Auge G, Cochaud C, Lespinasse F, N'Guyen K, de Septenville A, Brice A, Yu-Wai-Man P, Sesaki H, Pouget J, Paquis-Flucklinger V. A mitochondrial origin for frontotemporal dementia and amyotrophic lateral sclerosis through CHCHD10 involvement. Brain. 2014 Aug;137(Pt 8):2329-45
Müller K, Andersen PM, Hübers A, Marroquin N, Volk AE, Danzer KM, Meitinger T, Ludolph AC, Strom TM, Weishaupt JH. Two novel mutations in conserved codons indicate that CHCHD10 is a gene associated with motor neuron disease. Brain. 2014 Dec;137(Pt 12):e309. doi: 10.1093/brain/awu227. Epub 2014 Aug 11.
Johnson JO, Glynn SM, Gibbs JR, Nalls MA, Sabatelli M, Restagno G, Drory VE, Chiò A, Rogaeva E, Traynor BJ. Mutations in the CHCHD10 gene are a common cause of familial amyotrophic lateral sclerosis. Brain. 2014 Dec;137(Pt 12):e311. doi: 10.1093/brain/awu265. Epub 2014 Sep 26.
Abdulhag UN, Soiferman D, Schueler-Furman O, Miller C, Shaag A, Elpeleg O, Edvardson S, Saada A. Mitochondrial complex IV deficiency, caused by mutated COX6B1, is associated with encephalomyopathy, hydrocephalus and cardiomyopathy. Eur J Hum Genet. 2015 Feb;23(2):159-64. doi: 10.1038/ejhg.2014.85. Epub 2014 Apr 30.
Ghosh A, Trivedi PP, Timbalia SA, Griffin AT, Rahn JJ, Chan SS, Gohil VM. Copper supplementation restores cytochrome c oxidase assembly defect in a mitochondrial disease model of COA6 deficiency. Hum Mol Genet. 2014 Jul 1;23(13):3596-606. doi: 10.1093/hmg/ddu069. Epub 2014 Feb 18.
Baertling F, A M van den Brand M, Hertecant JL, Al-Shamsi A, P van den Heuvel L, Distelmaier F, Mayatepek E, Smeitink JA, Nijtmans LG, Rodenburg RJ. Mutations in COA6 cause cytochrome c oxidase deficiency and neonatal hypertrophic cardiomyopathy. Hum Mutat. 2015 Jan;36(1):34-8. doi: 10.1002/humu.22715. Epub 2014 Nov 18.
Jonckheere Al1, Huigsloot M, Lammens M, Jansen J, van den Heuvel LP, Spiekerkoetter U, von Kleist-Retzow JC, Forkink M, Koopman WJ, Szklarczyk R, Huynen MA, Fransen JA, Smeitink JA, Rodenburg RJ. Restoration of complex V deficiency caused by a novel deletion in the human TMEM70 gene normalizes mitochondrial morphology. Mitochondrion. 2011 Nov;11(6):954-63. doi: 10.1016/j.mito.2011.08.012. Epub 2011 Sep 14.
Logan CV, Szabadkai G, Sharpe JA, Parry DA, Torelli S, Childs AM, Kriek M, Phadke R, Johnson CA, Roberts NY, Bonthron DT, Pysden KA, Whyte T, Munteanu I, Foley AR, Wheway G, Szymanska K, Natarajan S, Abdelhamed ZA, Morgan JE, Roper H, Santen GW, Niks EH, van der Pol WL, Lindhout D, Raffaello A, De Stefani D, den Dunnen JT, Sun Y, Ginjaar I, Sewry CA, Hurles M, Rizzuto R; UK10K Consortium, Duchen MR, Muntoni F, Sheridan E. Loss-of-function mutations in MICU1 cause a brain and muscle disorder linked to primary alterations in mitochondrial calcium signaling. Nat Genet. 2014 Feb;46(2):188-93. doi: 10.1038/ng.2851. Epub 2013 Dec 15.
Vantroys E1, Larson A2, Friederich M2, Knight K2, Swanson MA2, Powell CA3, Smet J1, Vergult S4, De Paepe B1, Seneca S5, Roeyers H6, Menten B4, Minczuk M3, Vanlander A1, Van Hove J2, Van Coster R7. New insights into the phenotype of FARS2 deficiency. Mol Genet Metab. 2017 Dec;122(4):172-181. doi: 10.1016/j.ymgme.2017.10.004. Epub 2017 Oct 12.
Ferreira M1, Torraco A, Rizza T, Fattori F, Meschini MC, Castana C, Go NE, Nargang FE, Duarte M, Piemonte F, Dionisi-Vici C, Videira A, Vilarinho L, Santorelli FM, Carrozzo R, Bertini E. Progressive cavitating leukoencephalopathy associated with respiratory chain complex I deficiency and a novel mutation in NDUFS1. Neurogenetics. 2011 Feb;12(1):9-17. doi: 10.1007/s10048-010-0265-2. Epub 2011 Jan 4.
Tranebjaerg L, Hamel BC, Gabreels FJ, Renier WO, Van Ghelue M. A de novo missense mutation in a critical domain of the X-linked DDP gene causes the typical deafness-dystonia-optic atrophy syndrome. Eur J Hum Genet. 2000 Jun;8(6):464-7.
Six E1, Lagresle-Peyrou C1, Susini S1, De Chappedelaine C1, Sigrist N1, Sadek H1, Chouteau M1, Cagnard N2, Fontenay M3, Hermine O4, Chomienne C5, Reynier P6, Fischer A7, Andre-Schmutz I1, Gueguen N6, Cavazzana M8. AK2 deficiency compromises the mitochondrial energy metabolism required for differentiation of human neutrophil and lymphoid lineages. Cell Death Dis. 2015 Aug 13;6:e1856. doi: 10.1038/cddis.2015.211.
Weraarpachai W, Sasarman F, Nishimura T, Antonicka H, Auré K, Rötig A, Lombès A, Shoubridge EA. Mutations in C12orf62, a factor that couples COX I synthesis with cytochrome c oxidase assembly, cause fatal neonatal lactic acidosis. Am J Hum Genet. 2012 Jan 13;90(1):142-51. doi: 10.1016/j.ajhg.2011.11.027.
Haack TB, Rolinski B, Haberberger B, Zimmermann F, Schum J, Strecker V, Graf E, Athing U, Hoppen T, Wittig I, Sperl W, Freisinger P, Mayr JA, Strom TM, Meitinger T, Prokisch H. Homozygous missense mutation in BOLA3 causes multiple mitochondrial dysfunctions syndrome in two siblings. J Inherit Metab Dis. 2013 Jan;36(1):55-62. doi: 10.1007/s10545-012-9489-7. Epub 2012 May 5.
Scheper GC1, van der Klok T, van Andel RJ, van Berkel CG, Sissler M, Smet J, Muravina TI, Serkov SV, Uziel G, Bugiani M, Schiffmann R, Krageloh-Mann I, Smeitink JA, Florentz C, Van Coster R, Pronk JC, van der Knaap MS. Mitochondrial aspartyl-tRNA synthetase deficiency causes leukoencephalopathy with brain stem and spinal cord involvement and lactate elevation. Nat Genet. 2007 Apr;39(4):534-9. Epub 2007 Mar 25.
Tamiya G1, Makino S2, Hayashi M3, Abe A3, Numakura C3, Ueki M2, Tanaka A4, Ito C5, Toshimori K5, Ogawa N6, Terashima T6, Maegawa H6, Yanagisawa D7, Tooyama 17, Tada M8, Onodera O8, Hayasaka K9. A mutation of COX6A1 causes a recessive axonal or mixed form of Charcot-Marie-Tooth disease. Am J Hum Genet. 2014 Sep 4;95(3):294-300. doi: 10.1016/j.ajhg.2014.07.013. Epub 2014 Aug 21.
Cheng J, Zhu Y, He S, Lu Y, Chen J, Han B, Petrillo M, Wrzeszczynski KO, Yang S, Dai P, Zhai S, Han D, Zhang MQ, Li W, Liu X, Li H, Chen ZY, Yuan H. Functional mutation of SMAC/DIABLO, encoding a mitochondrial proapoptotic protein, causes human progressive hearing loss DFNA64. Am J Hum Genet. 2011 Jul 15;89(1):56-66. doi: 10.1016/j.ajhg.2011.05.027. Epub 2011 Jun 30.
Zeharia A1, Friedman JR2, Tobar A3, Saada A4, Konen O5, Fellig Y6, Shaag A4, Nunnari J2, Elpeleg O4. Mitochondrial hepato-encephalopathy due to deficiency of QIL1/MIC13 (C19orf70), a MICOS complex subunit.Eur J Hum Genet. 2016 Dec;24(12):1778-1782. doi: 10.1038/ejhg.2016.83. Epub 2016 Aug 3.
Ostergaard E1, Rodenburg RJ, van den Brand M, Thomsen LL, Duno M, Batbayli M, Wibrand F, Nijtmans L. Respiratory chain complex I deficiency due to NDUFA12 mutations as a new cause of Leigh syndrome. J Med Genet. 2011 Nov;48(11):737-40. doi: 10.1136/jmg.2011.088856. Epub 2011 May 26.
Dunning CJ1, McKenzie M, Sugiana C, Lazarou M, Silke J, Connelly A, Fletcher JM, Kirby DM, Thorburn DR, Ryan MT. Human CIA30 is involved in the early assembly of mitochondrial complex I and mutations in its gene cause disease. EMBO J. 2007 Jul 11;26(13):3227-37. Epub 2007 Jun 7.
Gerards M1, van den Bosch BJ, Danhauser K, Serre V, van Weeghel M, Wanders RJ, Nicolaes GA, Sluiter W, Schoonderwoerd K, Scholte HR, Prokisch H, Rötig A, de Coo IF, Smeets HJ. Riboflavin-responsive oxidative phosphorylation complex I deficiency caused by defective ACAD9: new function for an old gene. Brain. 2011 Jan;134(Pt 1):210-9. doi: 10.1093/brain/awq273. Epub 2010 Oct 7.
Assereto S1, Robbiano A, Di Rocco M, Rossi A, Cassandrini D, Panicucci C, Brigati G, Biancheri R, Bruno C, Minetti C, Trucks H, Sander T, Zara F, Gazzerro E. Functional characterization of the c.462delA mutation in the NDUFS4 subunit gene of mitochondrial complex I. Clin Genet. 2014 Jul;86(1):99-101. doi: 10.1111/cge.12248. Epub 2013 Sep 11.
Kohda M1, Tokuzawa Y2, Kishita Y2, Nyuzuki H2, Moriyama Y2,3, Mizuno Y2, Hirata T1, Yatsuka Y2, Yamashita-Sugahara Y2, Nakachi Y1, Kato H1,3, Okuda A3, Tamaru S2, Borna NN2, Banshoya K2,4, Aigaki T5, Sato-Miyata Y5, Ohnuma K5, Suzuki T6, Nagao A6, Maehata H6, Matsuda F7, Higasa K7, Nagasaki M8,9,10, Yasuda J8,9, Yamamoto M8,9, Fushimi T11, Shimura M11, Kaiho-Ichimoto K11, Harashima H12, Yamazaki T12, Mori M13, Murayama K11, Ohtake A12, Okazaki Y1,2. A Comprehensive Genomic Analysis Reveals the Genetic Landscape of Mitochondrial Respiratory Chain Complex Deficiencies. PLoS Genet. 2016 Jan 7;12(1):e1005679. doi: 10.1371/journal.pgen.1005679. eCollection 2016 Jan.
McKenzie M, Tucker EJ, Compton AG, Lazarou M, George C, Thorburn DR, Ryan MT. Mutations in the gene encoding C8orf38 block complex I assembly by inhibiting production of the mitochondria-encoded subunit ND1. J Mol Biol. 2011 Dec 2;414(3):413-26. doi: 10.1016/j.jmb.2011.10.012. Epub 2011 Oct 14.
Melchionda L, Haack TB, Hardy S, Abbink TE, Fernandez-Vizarra E, Lamantea E, Marchet S, Morandi L, Moggio M, Carrozzo R, Torraco A, Diodato D, Strom TM, Meitinger T, Tekturk P, Yapici Z, Al-Murshedi F, Stevens R, Rodenburg RJ, Lamperti C, Ardissone A, Moroni I, Uziel G, Prokisch H, Taylor RW, Bertini E, van der Knaap MS, Ghezzi D, Zeviani M. Mutations in APOPT1, encoding a mitochondrial protein, cause cavitating leukoencephalopathy with cytochrome c oxidase deficiency. Am J Hum Genet. 2014 Sep 4;95(3):315-25. doi: 10.1016/j.ajhg.2014.08.003. Epub 2014 Aug 28.
Eid N1, Ito Y, Maemura K, Otsuki Y. Elevated autophagic sequestration of mitochondria and lipid droplets in steatotic hepatocytes of chronic ethanol-treated rats: an immunohistochemical and electron microscopic study. J Mol Histol. 2013 Jun;44(3):311-26. doi: 10.1007/s10735-013-9483-x. Epub 2013 Feb 1.
Mayr JA, Havlícková V, Zimmermann F, Magler I, Kaplanová V, Jesina P, Pecinová A, Nusková H, Koch J, Sperl W, Houstek J. Mitochondrial ATP synthase deficiency due to a mutation in the ATP5E gene for the F1 epsilon subunit. Hum Mol Genet. 2010 Sep 1;19(17):3430-9. doi: 10.1093/hmg/ddq254. Epub 2010 Jun 21.
Meulemans A1, Seneca S, Pribyl T, Smet J, Alderweirldt V, Waeytens A, Lissens W, Van Coster R, De Meirleir L, di Rago JP, Gatti DL, Ackerman SH.J Biol Chem. Defining the pathogenesis of the human Atp12p W94R mutation using a Saccharomyces cerevisiae yeast model. 2010 Feb 5;285(6):4099-109. doi: 10.1074/jbc.M109.046920. Epub 2009 Nov 20.
Coenen MJ1, van den Heuvel LP, Ugalde C, Ten Brinke M, Nijtmans LG, Trijbels FJ, Beblo S, Maier EM, Muntau AC, Smeitink JA. Cytochrome c oxidase biogenesis in a patient with a mutation in COX10 gene. Ann Neurol. 2004 Oct;56(4):560-4.
Antonicka H1, Leary SC, Guercin GH, Agar JN, Horvath R, Kennaway NG, Harding CO, Jaksch M, Shoubridge EA. Hum Mol Genet. Mutations in COX10 result in a defect in mitochondrial heme A biosynthesis and account for multiple, early-onset clinical phenotypes associated with isolated COX deficiency.2003 Oct 15;12(20):2693-702. Epub 2003 Aug 19.
Szklarczyk R, Wanschers BF, Nijtmans LG, Rodenburg RJ, Zschocke J, Dikow N, van den Brand MA, Hendriks-Franssen MG, Gilissen C, Veltman JA, Nooteboom M, Koopman WJ, Willems PH, Smeitink JA, Huynen MA, van den Heuvel LP. A mutation in the FAM36A gene, the human ortholog of COX20, impairs cytochrome c oxidase assembly and is associated with ataxia and muscle hypotonia. Hum Mol Genet. 2013 Feb 15;22(4):656-67. doi: 10.1093/hmg/dds473. Epub 2012 Nov 2.
Stiburek L1, Vesela K, Hansikova H, Hulkova H, Zeman J. Loss of function of Sco1 and its interaction with cytochrome c oxidase. Am J Physiol Cell Physiol. 2009 May;296(5):C1218-26. doi: 10.1152/ajpcell.00564.2008. Epub 2009 Mar 18.
Valnot I, Osmond S, Gigarel N, Mehaye B, Amiel J, Cormier-Daire V, Munnich A, Bonnefont JP, Rustin P, Rötig A. Mutations of the SCO1 gene in mitochondrial cytochrome c oxidase deficiency with neonatal-onset hepatic failure and encephalopathy. Am J Hum Genet. 2000 Nov;67(5):1104-9. Epub 2000 Sep 28.
Stiburek L1, Vesela K, Hansikova H, Hulkova H, Zeman J. Am J Physiol Cell Physiol. 2009 Loss of function of Sco1 and its interaction with cytochrome c oxidase. May;296(5):C1218-26. doi: 10.1152/ajpcell.00564.2008. Epub 2009 Mar 18.
Gaignard P1, Menezes M, Schiff M, Bayot A, Rak M, Ogier de Baulny H, Su CH, Gilleron M, Lombes A, Abida H, Tzagoloff A, Riley L, Cooper ST, Mina K, Sivadorai P, Davis MR, Allcock RJ, Kresoje N, Laing NG, Thorburn DR, Slama A, Christodoulou J, Rustin P. Mutations in CYC1, encoding cytochrome c1 subunit of respiratory chain complex III, cause insulin-responsive hyperglycemia. Am J Hum Genet. 2013 Aug 8;93(2):384-9. doi: 10.1016/j.ajhg.2013.06.015. Epub 2013 Aug 1.
Antonicka H, Leary SC, Guercin GH, Agar JN, Horvath R, Kennaway NG, Harding CO, Jaksch M, Shoubridge EA. Mutations in COX10 result in a defect in mitochondrial heme A biosynthesis and account for multiple, early-onset clinical phenotypes associated with isolated COX deficiency. Hum Mol Genet. 2003 Oct 15;12(20):2693-702. Epub 2003 Aug 19.
Ghezzi D1, Saada A, D'Adamo P, Fernandez-Vizarra E, Gasparini P, Tiranti V, Elpeleg O, Zeviani M. FASTKD2 nonsense mutation in an infantile mitochondrial encephalomyopathy associated with cytochrome c oxidase deficiency. Am J Hum Genet. 2008 Sep;83(3):415-23. doi: 10.1016/j.ajhg.2008.08.009. Epub 2008 Sep 4.
Dallabona C1, Abbink TE2, Carrozzo R3, Torraco A3, Legati A4, van Berkel CG2, Niceta M5, Langella T4, Verrigni D3, Rizza T3, Diodato D3, Piemonte F3, Lamantea E4, Fang M6, Zhang J6, Martinelli D7, Bevivino E7, Dionisi-Vici C7, Vanderver A8, Philip SG9, Kurian MA10, Verma IC11, Bijarnia-Mahay S11, Jacinto S12, Furtado F13, Accorsi P14, Ardissone A15, Moroni 115, Ferrero I1, Tartaglia M5, Goffrini P1, Ghezzi D16, van der Knaap MS17, Bertini E18. LYRM7 mutations cause a multifocal cavitating leukoencephalopathy with distinct MRI appearance. Brain. 2016 Mar;139(Pt 3):782-94. doi: 10.1093/brain/awv392. Epub 2016 Jan 29.
Hoefs SJ, Dieteren CE, Distelmaier F, Janssen RJ, Epplen A, Swarts HG, Forkink M, Rodenburg RJ, Nijtmans LG, Willems PH, Smeitink JA, van den Heuvel LP. NDUFA2 complex I mutation leads to Leigh disease. Am J Hum Genet. 2008 Jun;82(6):1306-15. doi: 10.1016/j.ajhg.2008.05.007.
Ugalde C1, Janssen RJ, van den Heuvel LP, Smeitink JA, Nijtmans LG. Differences in assembly or stability of complex I and other mitochondrial OXPHOS complexes in inherited complex I deficiency. Hum Mol Genet. 2004 Mar 15;13(6):659-67. Epub 2004 Jan 28.
Varghese J, Ayala-Ramirez M, Rich T, Rohren E, Rao P, Jimenez C. Novel germline SDHD mutation: diagnosis and implications to the patient. Fam Cancer. 2011 Jun;10(2):365-71. doi: 10.1007/s10689-011-9421-6.
Kerscher S1, Benit P, Abdrakhmanova A, Zwicker K, Rais I, Karas M, Rustin P, Brandt U. Processing of the 24 kDa subunit mitochondrial import signal is not required for assembly of functional complex I in Yarrowia lipolytica. Eur J Biochem. 2004 Sep;271(17):3588-95.
Kevelam SH, Rodenburg RJ, Wolf NI, Ferreira P, Lunsing RJ, Nijtmans LG, Mitchell A, Arroyo HA, Rating D, Vanderver A, van Berkel CG, Abbink TE, Heutink P, van der Knaap MS. NUBPL mutations in patients with complex I deficiency and a distinct MRI pattern. Neurology. 2013 Apr 23;80(17):1577-83. doi: 10.1212/WNL.0b013e31828f1914. Epub 2013 Apr 3.
Tucker EJ, Mimaki M, Compton AG, McKenzie M, Ryan MT, Thorburn DR. Next-generation sequencing in molecular diagnosis: NUBPL mutations highlight the challenges of variant detection and interpretation. Hum Mutat. 2012 Feb;33(2):411-8. doi: 10.1002/humu.21654. Epub 2011 Dec 22. Loeffen J, Smeitink J, Triepels R, Smeets R, Schuelke M, Sengers R, Trijbels F, Hamel B, Mullaart R, van den Heuvel L. The first nuclear-encoded complex I mutation in a patient with Leigh syndrome. Am J Hum Genet. 1998 Dec;63(6): 1598-608.
Procaccio V, Wallace DC. Late-onset Leigh syndrome in a patient with mitochondrial complex I NDUFS8 mutations. Neurology. 2004 May 25;62(10):1899-901
Calvo SE1, Compton AG, Hershman SG, Lim SC, Lieber DS, Tucker EJ, Laskowski A, Garone C, Liu S, Jaffe DB, Christodoulou J, Fletcher JM, Bruno DL, Goldblatt J, Dimauro S, Thorburn DR, Mootha VK. Molecular diagnosis of infantile mitochondrial disease with targeted next-generation sequencing. Sci Transl Med. 2012 Jan 25;4(118):118ra10. doi: 10.1126/scitranslmed.3003310.
Haack TB1, Haberberger B, Frisch EM, Wieland T, luso A, Gorza M, Strecker V, Graf E, Mayr JA, Herberg U, Hennermann JB, Klopstock T, Kuhn KA, Ahting U, Sperl W, Wilichowski E, Hoffmann GF, Tesarova M, Hansikova H, Zeman J, Plecko B, Zeviani M, Wittig I, Strom TM, Schuelke M, Freisinger P, Meitinger T, Prokisch H. Molecular diagnosis in mitochondrial complex I deficiency using exome sequencing. J Med Genet. 2012 Apr;49(4):277-83. doi: 10.1136/jmedgenet-2012-100846.
Fernandez-Moreira D1, Ugalde C, Smeets R, Rodenburg RJ, Lopez-Laso E, Ruiz-Falco ML, Briones P, Martin MA, Smeitink JA, Arenas J. X-linked NDUFA1 gene mutations associated with mitochondrial encephalomyopathy. Ann Neurol. 2007 Jan;61(1):73-83.
Potluri P, Davila A, Ruiz-Pesini E, Mishmar D, O'Hearn S, Hancock S, Simon M, Scheffler IE, Wallace DC, Procaccio V. A novel NDUFA1 mutation leads to a progressive mitochondrial complex I-specific neurodegenerative disease. Mol Genet Metab. 2009 Apr;96(4):189-95. doi: 10.1016/j.ymgme.2008.12.004. Epub 2009 Jan 29.
Saada A1, Vogel RO, Hoefs SJ, van den Brand MA, Wessels HJ, Willems PH, Venselaar H, Shaag A, Barghuti F, Reish O, Shohat M, Huynen MA, Smeitink JA, van den Heuvel LP, Nijtmans LG. Mutations in NDUFAF3 (C3ORF60), encoding an NDUFAF4 (C6ORF66)-interacting complex I assembly protein, cause fatal neonatal mitochondrial disease. Am J Hum Genet. 2009 Jun;84(6):718-27. doi: 10.1016/j.ajhg.2009.04.020. Epub 2009 May 21.
Kirby DM1, Salemi R, Sugiana C, Ohtake A, Parry L, Bell KM, Kirk EP, Boneh A, Taylor RW, Dahl HH, Ryan MT, Thorburn DR. NDUFS6 mutations are a novel cause of lethal neonatal mitochondrial complex I deficiency. J Clin Invest. 2004 Sep;114(6):837-45.
Spiegel R1, Shaag A, Mandel H, Reich D, Penyakov M, Hujeirat Y, Saada A, Elpeleg O, Shalev SA. Mutated NDUFS6 is the cause of fatal neonatal lactic acidemia in Caucasus Jews. Eur J Hum Genet. 2009 Sep;17(9):1200-3. doi: 10.1038/ejhg.2009.24. Epub 2009 Mar 4.
Benit P1, Slama A, Cartault F, Giurgea I, Chretien D, Lebon S, Marsac C, Munnich A, Rötig A, Rustin P. Mutant NDUFS3 subunit of mitochondrial complex I causes Leigh syndrome. J Med Genet. 2004 Jan;41(1):14-7.
Haack TB1, Haberberger B, Frisch EM, Wieland T, luso A, Gorza M, Strecker V, Graf E, Mayr JA, Herberg U, Hennermann JB, Klopstock T, Kuhn KA, Ahting U, Sperl W, Wilichowski E, Hoffmann GF, Tesarova M, Hansikova H, Zeman J, Plecko B, Zeviani M, Wittig I, Strom TM, Schuelke M, Freisinger P, Meitinger T, Prokisch H. Molecular diagnosis in mitochondrial complex I deficiency using exome sequencing. J Med Genet. 2012 Apr;49(4):277-83. doi: 10.1136/jmedgenet-2012-100846.
van Rahden VA1, Fernandez-Vizarra E2, Alawi M3, Brand K1, Fellmann F4, Horn D5, Zeviani M2, Kutsche K6. Mutations in NDUFB11, encoding a complex I component of the mitochondrial respiratory chain, cause microphthalmia with linear skin defects syndrome. Am J Hum Genet. 2015 Apr 2;96(4):640-50. doi: 10.1016/j.ajhg.2015.02.002. Epub 2015 Mar 12.
Levitas A, Muhammad E, Harel G, Saada A, Caspi VC, Manor E, Beck JC, Sheffield V, Parvari R. Familial neonatal isolated cardiomyopathy caused by a mutation in the flavoprotein subunit of succinate dehydrogenase. Eur J Hum Genet. 2010 Oct;18(10):1160-5. doi: 10.1038/ejhg.2010.83. Epub 2010 Jun 16.
Bourgeron T, Rustin P, Chretien D, Birch-Machin M, Bourgeois M, Viegas-Péquignot E, Munnich A, Rötig A. Mutation of a nuclear succinate dehydrogenase gene results in mitochondrial respiratory chain deficiency. Nat Genet. 1995 Oct; 11(2):144-9.
Parfait B, Chretien D, Rötig A, Marsac C, Munnich A, Rustin P. Compound heterozygous mutations in the flavoprotein gene of the respiratory chain complex II in a patient with Leigh syndrome. Hum Genet. 2000 Feb;106(2):236-43.
Ghezzi D, Goffrini P, Uziel G, Horvath R, Klopstock T, Lochmüller H, D'Adamo P, Gasparini P, Strom TM, Prokisch H, Invernizzi F, Ferrero I, Zeviani M. SDHAF1, encoding a LYR complex-II specific assembly factor, is mutated in SDH-defective infantile leukoencephalopathy. Nat Genet. 2009 Jun;41(6):654-6. doi: 10.1038/ng.378. Epub 2009 May 24.
Péquignot MO, Dey R, Zeviani M, Tiranti V, Godinot C, Poyau A, Sue C, Di Mauro S, Abitbol M, Marsac C. Mutations in the SURF1 gene associated with Leigh syndrome and cytochrome C oxidase deficiency. Hum Mutat. 2001 May;17(5):374-81.
Sanchez-Caballero L1, Ruzzenente B2, Bianchi L2, Assouline Z3, Barcia G3, Metodiev MD2, Rio M3, Funalot B4, van den Brand MA1, Guerrero-Castillo S1, Molenaar JP5, Koolen D6, Brandt U1, Rodenburg RJ1, Nijtmans LG7, Rötig A8. Mutations in Complex I Assembly Factor TMEM126B Result in Muscle Weakness and Isolated Complex I Deficiency. Am J Hum Genet. 2016 Jul 7;99(1):208-16. doi: 10.1016/j.ajhg.2016.05.022. Epub 2016 Jun 30.
Nogueira C1, Barros J, Sá MJ, Azevedo L, Taipa R, Torraco A, Meschini MC, Verrigni D, Nesti C, Rizza T, Teixeira J, Carrozzo R, Pires MM, Vilarinho L, Santorelli FM. Novel TTC19 mutation in a family with severe psychiatric manifestations and complex III deficiency. Neurogenetics. 2013 May;14(2):153-60. doi: 10.1007/s10048-013-0361-1. Epub 2013 Mar 28.
Kremer LS1,2, Bader DM3,4, Mertes C3, Kopajtich R1,2, Pichler G5, luso A1,2, Haack TB1,2, Graf E1,2, Schwarzmayr T1,2, Terrile C1, Koňaříková E1,2, Repp B1,2, Kastenmüller G6, Adamski J7, Lichtner P1, Leonhardt C8, Funalot B9, Donati A10, Tiranti V11, Lombes A12,13,14, Jardel C12, 15, Glaser D16, Taylor RW17, Ghezzi D11, Mayr JA18, Rötig A9, Freisinger P19, Distelmaier F20, Strom TM1,2, Meitinger T1,2, Gagneur J3,4, Prokisch H1,2. Genetic diagnosis of Mendelian disorders via RNA sequencing. Nat Commun. 2017 Jun 12;8:15824. doi: 10.1038/ncomms15824.
Spiegel R, Khayat M, Shalev SA, Horovitz Y, Mandel H, Hershkovitz E, Barghuti F, Shaag A, Saada A, Korman SH, Elpeleg O, Yatsiv I. TMEM70 mutations are a common cause of nuclear encoded ATP synthase assembly defect: further delineation of a new syndrome. J Med Genet. 2011 Mar;48(3):177-82. doi: 10.1136/jmg.2010.084608. Epub 2010 Dec 8.
Cízková A1, Stránecký V, Mayr JA, Tesarová M, Havlícková V, Paul J, Ivánek R, Kuss AW, Hansíková H, Kaplanová V, Vrbacký M, Hartmannová H, Nosková L, Honzík T, Drahota Z, Magner M, Hejzlarová K, Sperl W, Zeman J, Houstek J, Kmoch S. TMEM70 mutations cause isolated ATP synthase deficiency and neonatal mitochondrial encephalocardiomyopathy. Nat Genet. 2008 Nov;40(11):1288-90. doi: 10.1038/ng.246. Epub 2008 Oct 26.
Tucker EJ1, Wanschers BF2, Szklarczyk R3, Mountford HS1, Wijeyeratne XW4, van den Brand MA5, Leenders AM5, Rodenburg RJ5, Reljić B4, Compton AG1, Frazier AE1, Bruno DL6, Christodoulou J7, Endo H8, Ryan MT9, Nijtmans LG5, Huynen MA3, Thorburn DR10. Mutations in the UQCC1-interacting protein, UQCC2, cause human complex III deficiency associated with perturbed cytochrome b protein expression. PLoS Genet. 2013;9(12):e1004034. doi: 10.1371/journal.pgen.1004034. Epub 2013 Dec 26.
Wanschers BF1, Szklarczyk R2, van den Brand MA3, Jonckheere A3, Suijskens J4, Smeets R4, Rodenburg RJ3, Stephan K5, Helland IB6, Elkamil A7, Rootwelt T8, Ott M5, van den Heuvel L9, Nijtmans LG3, Huynen MA10. A mutation in the human CBP4 ortholog UQCC3 impairs complex III assembly, activity and cytochrome b stability. Hum Mol Genet. 2014 Dec 1;23(23):6356-65. doi: 10.1093/hmg/ddu357. Epub 2014 Jul 9.
Miyake N1, Yano S, Sakai C, Hatakeyama H, Matsushima Y, Shiina M, Watanabe Y, Bartley J, Abdenur JE, Wang RY, Chang R, Tsurusaki Y, Doi H, Nakashima M, Saitsu H, Ogata K, Goto Y, Matsumoto N. Mitochondrial complex III deficiency caused by a homozygous UQCRC2 mutation presenting with neonatal-onset recurrent metabolic decompensation. Hum Mutat. 2013 Mar;34(3):446-52. doi: 10.1002/humu.22257. Epub 2013 Jan 29.
Gaignard P1, Eyer D2, Lebigot E1, Oliveira C1, Therond P1, Boutron A1, Slama A1. UQCRC2 mutation in a patient with mitochondrial complex III deficiency causing recurrent liver failure, lactic acidosis and hypoglycemia. J Hum Genet. 2017 Jul;62(7):729-731. doi: 10.1038/jhg.2017.22. Epub 2017 Mar 9.
Barel O1, Shorer Z, Flusser H, Ofir R, Narkis G, Finer G, Shalev H, Nasasra A, Saada A, Birk OS. Mitochondrial complex III deficiency associated with a homozygous mutation in UQCRQ. Am J Hum Genet. 2008 May;82(5):1211-6. doi: 10.1016/j.ajhg.2008.03.020. Epub 2008 Apr 24.
Haut S1, Brivet M, Touati G, Rustin P, Lebon S, Garcia-Cazorla A, Saudubray JM, Boutron A, Legrand A, Slama A. A deletion in the human QP-C gene causes a complex III deficiency resulting in hypoglycaemia and lactic acidosis. Hum Genet. 2003 Jul; 113(2): 118-22. Epub 2003 Apr 23.
Kozjak-Pavlovic V, Prell F, Thiede B, Götz M, Wosiek D, Ott C, Rudel T. C1orf163/RESA1 is a novel mitochondrial intermembrane space protein connected to respiratory chain assembly. J Mol Biol. 2014 Feb 20;426(4):908-20. doi: 10.1016/j.jmb.2013.12.001. Epub 2013 Dec 9.
Bragoszewski P, Wasilewski M, Sakowska P, Gornicka A, Böttinger L, Qiu J, Wiedemann N, Chacinska A. Retro-translocation of mitochondrial intermembrane space proteins. Proc Natl Acad Sci U S A. 2015 Jun 23;112(25):7713-8. doi: 10.1073/pnas.1504615112. Epub 2015
Huang X, Dixit VM. Drugging the undruggables: exploring the ubiquitin system for drug development. Cell Res. 2016 Apr;26(4):484-98. doi: 10.1038/cr.2016.31. Epub 2016 Mar 22.
Roesch K1, Curran SP, Tranebjaerg L, Koehler CM. Human deafness dystonia syndrome is caused by a defect in assembly of the DDP1/TIMM8a-TIMM13 complex. Hum Mol Genet. 2002 Mar 1;11(5):477-86.
Tranebjaerg L, Hamel BC, Gabreels FJ, Renier WO, Van Ghelue M. A de novo missense mutation in a critical domain of the X-linked DDP gene causes the typical deafness-dystonia-optic atrophy syndrome. Eur J Hum Genet. 2000 Jun;8(6):464-7.
Stroud DA, Maher MJ, Lindau C, Vögtle FN, Frazier AE, Surgenor E, Mountford H, Singh AP, Bonas M, Oeljeklaus S, Warscheid B, Meisinger C, Thorburn DR, Ryan MT. COA6 is a mitochondrial complex IV assembly factor critical for biogenesis of mtDNA-encoded COX2. Hum Mol Genet. 2015 Oct 1;24(19):5404-15. doi: 10.1093/hmg/ddv265. Epub 2015 Jul 9.
Koehler CM, Leuenberger D, Merchant S, Renold A, Junne T, Schatz G. Human deafness dystonia syndrome is a mitochondrial disease. Proc Natl Acad Sci U S A. 1999 Mar 2;96(5):2141-6.
Tatyana A. Grigoreva, Vyacheslav G. Tribulovich, Alexander V. Garabadzhiu. The 26S proteasome is a multifaceted target for anti-cancer therapies Gerry Melino , Nickolai A. Barlev Oncotarget, Vol. 6, No. 28
Sakowska, P., Jans, D.C., Mohanraj, K., Riedel, D., Jakobs, S., Chacinska, A. The Oxidation Status of Mic19 Regulates MICOS Assembly. Mol Cell Biol. 35(24), 4222-37 (2015)
Fischer, M., Horn, S., Belkacemi, A., Kojer, K., Petrungaro, C., Habich, M., Ali, M., Küttner, V., Bien, M., Kauff, F., Dengjel, J., Herrmann, J.M., Riemer, J. Protein import and oxidative folding in the mitochondrial intermembrane space of intact mammalian cells. Mol Biol Cell. 24(14): 2160-70 (2013)

## Claims

1. An inhibitor of proteolytic activity for use in a method of treating or preventing a mitochondriopathy caused by at least one genetic mutation.

2. The inhibitor of proteolytic activity according to any of the preceding claims, wherein the mitochondriopathy is caused by at least one mutation in a nuclear gene.

3. The inhibitor of proteolytic activity according to any of the preceding claims, wherein the nuclear gene encodes a mitochondrial protein selected from a mitochondrial outer membrane protein, a mitochondrial intermembrane space protein, a mitochondrial inner membrane protein, a respiratory chain complex protein, and/or a mitochondrial matrix protein.

4. The inhibitor of proteolytic activity according to claim 3, wherein the protein is a MIA40 protein substrate.

5. The inhibitor of proteolytic activity according to any of the preceding claims, wherein the nuclear gene encodes a protein that is involved in mitochondrial biogenesis and/or function.

6. The inhibitor of proteolytic activity according to any of claims 3 to 5, wherein the protein is selected from RESA1, FOXRED1, augmenter of liver regeneration protein (ALR), coiled-coil-helix-coiled-coil-helix domain-containing protein 10 (CHCHD10), cytochrome c oxidase, subunit 6B1 (COX6B1), cytochrome c oxidase assembly factor 6 (COA6), cytochrome c oxidase assembly factor 5 (COA5), mitochondrial calcium uptake protein 1 (MICU1), NADH -ubiquinone oxidoreductase 1 alpha subcomplex 8 (NDUFA8), NADH-ubiquinone oxidoreductase 1 beta subcomplex 10 (NDUFB10), NADH-ubiquinone oxidoreductase fe-s protein 1 (NDUFS1) translocase of inner mitochondrial membrane 8 (TIMM8A), adenylate kinase 2 (AK2), cytochrome c oxidase subunit 8A (COX8A), cytochrome C oxidase assembly factor (COX14), NADH-ubiquinone oxidoreductase 1 beta subcomplex 9 (NDUFB9), cytochrome c oxidase assembly factor (COX15), cytochrome C oxidase subunit 6A1 (COX6A1), DIABLO/SMAC, FAD-dependent oxidoreductase domain-containing protein 1 (FOXRED1), MIC13, NADH -ubiquinone oxidoreductase 1 alpha subcomplex 12 (NDUFA12), NADH dehydrogenase (ubiquinone) complex I assembly factor 1 (NDUFAF1), NADH dehydrogenase (ubiquinone) complex I assembly factor 4 (NDUFAF4), NADH-ubiquinone oxidoreductase Fe-S protein 4 (NDUFS4), NADH dehydrogenase (ubiquinone) complex I assembly factor 6 (NDUFAF6) succinate dehydrogenase complex, subunit D, integral membrane protein; (SDHD), sulfite oxidase (SUOX), tetratricopeptide repeat domain-containing protein 19 (TTC19), mitochondrial apoptogenic protein 1 (APOPT1), H+ transporting ATP synthase mitochondrial F1 complex alpha subunit 1 (ATP5A1), H+ transporting ATP synthase mitochondrial F1 complex epsilon subunit (ATP5E), Atp Synthase, mitochondrial F1 complex assembly factor 2 (ATPAF2), cytochrome c oxidase assembly factor 10 (COX10), cytochrome c oxidase assembly factor 20 (COX20), cytochrome c oxidase assembly protein (SCO1), cytochrome C1 (CYC1), fast kinase domains 2 (FASTKD2), Lyr motif-containing protein 7 (LYRM7), NADH dehydrogenase 1 alpha subcomplex 11 (NDUFA11), NADH-ubiquinone oxidoreductase 1 alpha subcomplex 2 (NDUFA2), NADH-ubiquinone oxidoreductase 1 alpha subcomplex 9 (NDUFA9), NADH-ubiquinone oxidoreductase Fe-S protein 2 (NDUFS2), NADH-ubiquinone oxidoreductase flavoprotein 1 (NDUFV1), nucleotide-binding protein-like protein (NUBPL), NADH-ubiquinone oxidoreductase Fe-S protein 8 (NDUFS8), NADH-ubiquinone oxidoreductase beta subcomplex 3 (NDUFB3), NADH-ubiquinone oxidoreductase 1 alpha subcomplex 1 (NDUFA1), NADH dehydrogenase (ubiquinone) Complex I assembly factor 3 (NDUFAF3), NADH-ubiquinone oxidoreductase Fe-S protein 6 (NDUFS6), NADH dehydrogenase 1 beta subcomplex 11 (NDUFB11), succinate dehydrogenase complex subunit a flavoprotein (SDHA), succinate dehydrogenase complex assembly factor 1 (SDHAF1), SURFEIT 1 (SURF1), transmembrane protein 126B (TMEM126B), tetratricopeptide repeat domain-containing protein 19 (TTC19), translational activator of mitochondrially encoded cytochrome c oxidase subunit I (TACO1), translocase of inner mitochondrial membrane domain-containing protein 1 (TIMMDC1), transmembrane protein 70 (TMEM70), ubiquinol-cytochrome c reductase complex assembly factor 2 (UQCC2), ubiquinol-cytochrome c reductase complex assembly factor 3 (UQCC3), ubiquinol-cytochrome c reductase core protein II (UQCRC2), ubiquinol-cytochrome c reductase complex III subunit VII (UQCRQ), and/or ubiquinol-cytochrome c reductase-binding protein (UQCRB).

7. The inhibitor of proteolytic activity according to any of the preceding claims, wherein the mutation results in the substitution, deletion, and/or insertion of at least one amino acid in a mitochondrial protein.

8. The inhibitor of proteolytic activity according to claims 4 to 7, wherein the mutation results in at least a partial misfolding or instability of the protein.

9. The inhibitor of proteolytic activityaccording to any of the preceding claims, wherein the mutation is a mutation causing a substitution of amino acid 137 or a deletion of the 47 amino acids encoded by exon 2 of RESA1.

10. The inhibitor of proteolytic activity according to any of the preceding claims, wherein the inhibitor inhibits the proteasome, preferably 26S proteasome.

11. The inhibitor of proteolytic activity according to any of the preceding claims, wherein the proteasome inhibitor is a ubiquitin ligase inhibitor, a ubiquitin specific peptidase inhibitor, a ubiquitin-like modifier activating enzyme inhibitor, and/or a ubiquitin-specific protease inhibitor.

12. The inhibitor of proteolytic activity according to any of the preceding claims, wherein the proteasome inhibitor inhibits chymotrypsin-like and/or peptidylglutamyl peptide hydrolysing activity.

13. A inhibitor of proteolytic activity selected from bortezomib, carfilzomib or MG132 for use according to any of the preceding claims

14. The according to any of the preceding claims, wherein the mitochondriopathy is **characterized by** a decreased amount of at least one mitochondrial protein according to claims 4 to 7.

15. The inhibitor of proteolytic activity according to any of the preceding claims, wherein the mitochondriopathy is selected from mitochondrial myopathy, preferably progressive mitochondrial myopathy, cardiomyopathy, preferably dilated 1GG; mitochondrial myopathy with congenital cataract, hearing loss, and developmental delay, isolated autosomal dominant mitochondrial myopathy, mitochondrial myopathy with extrapyramidal signs, cardioencephalomyopathy, preferably fatal infantile cardioencephalomyopathy, frontotemporal dementia and/or amyotrophic lateral sclerosis 2, spinal muscular atrophy, preferably Jokela type spinal muscular atrophy, myopathy with extrapyramidal signs, mitochondrial complex I deficiency, mitochondrial complex IV deficiency, Mohr-Tranebjaerg syndrome, reticular dysgenesis, mitochondrial complex IV deficiency, Leigh syndrome due to cytochrome c oxidase deficiency, mitochondrial hepatoencephalopathy, Cowden syndrome 3, somatic merkel cell carcinoma, mitochondrial complex II deficiency, pagangliomas 1, preferably with or without deafness; paragangliomas 5, sulfate oxidase deficiency, mitochondrial complex III deficiency, preferably nuclear type 2; combined oxidative phosphorylation deficiency 22, Mitochondrial complex V deficiency, preferably nuclear type 1, 3 or 4; mitochondrial complex I deficiency, Leigh syndrome due to mitochondrial complex I deficiency, Leigh syndrome due to COX IV deficiency, and/or mitochondrial complex III deficiency, preferably nuclear type 2, 3, 4, 5, 7, 8, or 9.

16. A method for treating or preventing a mitochondriopathy caused by a genetic mutation by decreasing proteolytic activity within a cell, wherein the method comprises a step of decreasing the proteolytic activity within a cell.

17. The method according to claim 16, wherein the step of decreasing the proteolytic activity reduces or abolish the transcription or translation of a gene encoding a protease or a protein that otherwise contributes to, or regulates the activity of proteolytic activity within a cell.

18. The method according to claims 16 or 17, wherein the step of decreasing the proteolytic activity comprises subjecting a cell to an inhibitor of proteolytic activity.

19. The method according to claim 16, wherein the step of decreasing the proteolytic activity comprises a step of altering the genetic information within a cell.

20. The method according to claim 19, wherein the step of step of altering the genetic information comprises the disruption of at least one gene that contribute to the proteolytic activity within the cell, a mutation of said gene, or the disruption or mutation of regulatory elements, or wherein the step of step of altering the genetic information introduces genetic information that promotes the decrease of proteolytic activity by expressing inhibitory elements.
